# EUROPEAN PATENT APPLICATION

(11) **EP 2 082 766 A1**
(43) Date of publication of application: **29.07.2009**
(21) Application number: 08100879.9
(22) Date of filing: 24.01.2008
(51) Int. Cl.: A61M 15/00

(54) **Blister Strip Coil Forming**

(71) Applicant: Vectura Delivery Devices Limited, Chippenham, Wiltshire SN14 6FH (GB)
(72) Inventor: Gibbins, Graham, Foulsham, Norfolk NR20 5RW (GB); Evans, Peter, Cambridgeshire, CB6 2ST (GB); Sarkar, Matthew, Cambridge CB4 3JU (GB); Huxley, Stewart, Cambridge CB3 0QA (GB)
(74) Representative: Grey, Ian Michael

(57) **Abstract**

A method of coiling a strip of blisters for insertion into an inhalation device is disclosed. The method includes the steps of taking a preformed strip of blisters in which each blister contains a dose of medicament for inhalation by a user, manipulating the strip to impart a continuous curvature or a series of discrete creases to the strip; that allow the strip to form into a coiled shape. A strip which has been coiled according to the method is also disclosed, as is an inhalation device incorporating means for coiling a strip of blisters according to the invention.

## Description

The present invention relates to a method of coiling a strip having a number of blisters each of which contain a dose of medicament for inhalation by a user of an inhalation device in which the strip is located subsequent to being coiled. The invention also relates to a device for coiling such a strip and to an inhalation device incorporating a strip that has been coiled according to the method and/or using the device of the invention.

The present invention also relates to an inhalation device incorporating a device for coiling the used portion of the blister strip according to the invention, within the device.

Oral or nasal delivery of a medicament using an inhalation device is a particularly attractive method of drug administration as these devices are relatively easy for a patient to use discreetly and in public. As well as delivering medicament to treat local diseases of the airway and other respiratory problems, they have more recently also been used to deliver drugs to the bloodstream via the lungs thereby avoiding the need for hypodermic injections.

It is common for dry powder formulations to be pre-packaged in individual doses, usually in the form of capsules or blisters which each contain a single dose of the powder which has been accurately and consistently measured. A blister is generally cold formed from a ductile foil laminate or a plastics material and includes a puncturable or peelable lid which is heat-sealed around the periphery of the blister during manufacture and after introduction of the dose into the blister. A foil blister is preferred over a polymer blister or gelatine capsule as each dose is protected from the ingress of water and penetration of gases such as oxygen in addition to being shielded from light and UV radiation all of which can have a detrimental effect on the delivery characteristics of the inhaler if a dose becomes exposed to them. Therefore, a blister offers excellent environmental protection to each individual drug dose.

Inhalation devices that receive a blister pack comprising a number of blisters each of which contain a pre-metexed and individually packaged dose of the drug to be delivered are known. Actuation of the device causes a mechanism to breach or rupture a blister, such as by puncturing it or peeling the lid off, so that when the patient inhales, air is drawn through the blister entraining the dose therein that is then carried out of the blister through the device and via the patient's airway down into the lungs. Pressurized air or gas or other propellants may also be used to carry the dose out of the blister. Alternatively, the mechanism that punctures or opens the blister may also push or eject the dose out of the blister into a receptacle from which the dose may subsequently be inhaled.

It is advantageous for the inhaler to be capable of holding a number of doses to enable it to be used repeatedly over a period of time without the requirement to open and/or insert a blister into the device each time it is used. Therefore, many conventional devices include means for storing a number or strip of blisters each containing an individual dose of medicament. When a dose is to be inhaled, an indexing mechanism moves a previously emptied blister away from the opening mechanism so that a fresh one is moved into a position ready to be opened for inhalation of its contents.

An inhaler of the type described above is known from the Applicants own co-pending international application no. PCT/GB2004/004416 filed on 18th October 2004 and claiming priority from GB application no. 0324358.1 filed 17th October 2003. This international application has been published as WO 2005/037353 A1.

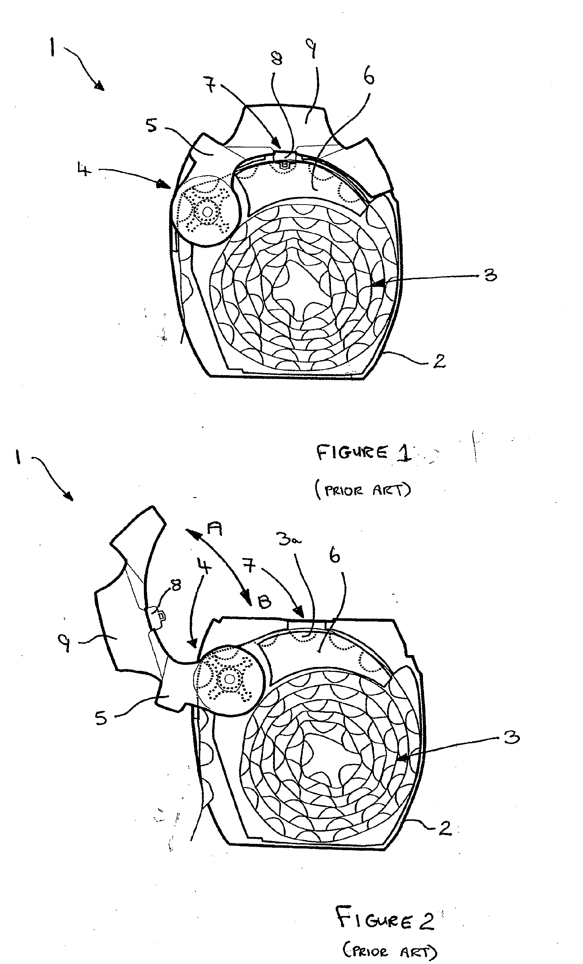

According to one embodiment described and claimed in WO 2005/037353 A1, and illustrated in Figures 1 and 2 of the accompanying drawings, an inhaler 1 has a housing 2 containing a coiled strip of blisters 3. An indexing mechanism 4 comprising a single actuating lever 5 unwinds the coil 3 one blister at a time so that they pass over a blister locator chassis 6 and successively through a blister piercing station 7, when the actuator 5 is pivoted in a direction indicated by arrow "A" in Figure 1b. The blister 3a located at the blister piercing station 7 on each movement of the actuator 5 is pierced on the return stroke of the actuator 5 (in the direction indicated by arrow "B" in Figure 1b) by piercing elements 8 on the actuator 5 itself so that, when a user inhales through a mouthpiece 9, an airflow is generated within the blister 3a to entrain the dose contained therein and carry it out of the blister 3a via the mouthpiece 9 and into the user's airway.

The coil formed from a strip of blisters, such as that shown in Figures 1 and 2, is conventionally formed by winding a substantially flat, elongate strip around a spindle in a winding rig. The resulting coil is then placed within the housing of the device. However, a problem with this known technique is that a coil wound in this way tends to naturally spring-open or radially expand due to resilience or residual spring tension in the resulting coil, unless held in place or constrained by some outside force acting upon it. As can be seen in Figures 1 and 2, the coil 3 has expanded or unwound so as to substantially occupy the entire space available within the housing, further expansion of the coil only being prevented or constrained by the walls of the device housing.

It will be appreciated that, as a result of the tendency for a conventional coil to naturally spring open or, at least, for a relatively tightly wound coil to expand so as to assume a more loosely wound coil, in the absence of any external forces, makes the process of removing a finished coil from a winding rig and that of placing it into the inhalation device during manufacture or assembly of such a device more complicated because the finished coil must be held in its closely wound state during this transfer step. It has also been found that a coil which expands due to its own natural resilience to fill the space that it occupies within the housing causes further coil control issues as it unwinds during use which can potentially lead to jamming of the strip or other device malfunctions. Therefore, it will be appreciated that it is desirable for the coil to remain tight and as compact as possible. It is also desirable for the coil to take up as little space as possible within the housing of a device

The present invention seeks to provide a method of coiling a strip of blisters which overcomes or substantially alleviates the disadvantages referred to in more detail above, so as to provide a compact coil that is resistant to expansion or uncoiling in the absence of external forces to keep it together in its initial, closely coiled, state.

The present invention also aims to provide a device for coiling a strip of blisters according to the method and, to an inhalation device incorporating such a coiled strip of unused blisters and to an inhalation device incorporating means, according to the method, of coiling up a strip of used blisters in the device after their use.

Although the unused blister strip can be coiled up as described prior to insertion into an inhalation device, it will be appreciated from a consideration of the conventional device shown in Figure 1, described in more detail above, that the coil is at least partially flattened or straightened out as it is guided over the blister piercing station, around the indexing wheel and down the side of the housing to a used blister containment region below the unused blister coil. This is acceptable in such a device in which the used blisters are not retained within the housing but pass out of the housing to enable a user to periodically detach and discard used portions of the strip. However, it is also desirable to provide a fully integrated device in which all the used blisters are retained within it. Various embodiments of such an inhalation device are known from the Applicant's own earlier European patent applications nos. 07111996.0 and 07111998.6. In the devices disclosed in these applications, the used blisters are coiled up within the housing. In such devices, to minimise overall device size, space freed up by the unused coil feeding out is reoccupied by the used coil feeding in. However, ability to do this cannot be maximised if, due to its resilience, the unused coil continuously expands as it feeds out to occupy available space.
It is therefore a further advantage of the method described above of preparing a coiled strip of blisters for insertion into an inhalation device that once inserted the unused part of the blister strip is neatly coiled up within the device without having any tendency to expand because it has been coiled in such a way that there is no residual spring tension remaining in the strip.

Furthermore, in addition to providing a closely wound coil of unused blisters, it is clearly advantageous to provide the device with a mechanism or device that encourages the used portion of the strip to coil up in the same or similar way as the unused portion has been coiled up. Therefore, although reference is made above to the preparation of a coiled strip of blisters ready for insertion into an inhalation device, it is also envisaged that the coil forming technique and components described herein can also be incorporated directly into an inhalation device so that the used part of the blister strip neatly coils up within the device as it is indexed through.

According to the present invention, there is provided a method of coiling a strip of blisters, the method including the steps of feeding a preformed strip of blisters around or through means that manipulates the strip so as to impart a continuous curvature or a series of discrete creases to the strip that allow the strip to assume a coiled shape.

The strip is manipulated so as to deform it. It is the plastic deformation of the strip which causes it to form a coiled shape. The deformations are either continuous in the sense that they extend along the length of the strip or, discrete individual regions of deformation are formed spaced from each other along the length of the strip.

In one embodiment, the method includes feeding said strip around a roller.

Preferably, the step of feeding the strip around a roller includes the step of feeding it between curved surfaces of two rollers positioned such that opposite sides of the strip are placed in compression and tension, respectively.

In one embodiment, the method includes the step of feeding the strip under two rollers having their axes parallel but spaced from each other and, over a third roller positioned between the two spaced rollers.

The method may include the step of positioning the third roller so that its axis is offset from a line extending between the axis of the first and second rollers so that a strip passing under the first roller must follow a curved path over the second roller.

The third roller may be configured so as to contact only the edges of the strip.

According to another embodiment, the step of feeding a preformed strip of blisters around or through means to manipulate the strip comprises feeding said strip through means to impart a series of discrete folds, creases or crimps extending transversely across the strip and allowing the strip to naturally assume a coiled shape due to said folds.

Although the curvature imparted to the strip can be continuous in the sense that the strip assumes an arcuate or naturally curved shape extending in a longitudinal direction, the same effect can also be achieved by imparting a series of discrete folds or creases across the strip, each fold being spaced from each other along its length. Even though the portions of the strip between folds are kept relatively straight, the repeated folds results in the strip forming itself into a coiled shape.

In one embodiment, creases or crimps can be imparted between the blisters of the strip, preferably all the blisters. However, it is also envisaged that they may be imparted to the strip between groups of blisters.

In one embodiment, said means comprises shaped jaws and the method includes controlling the jaws to close on a strip so that a fold is imparted to the strip by the jaws, the method further including opening the jaws to allow the strip to be fed between the jaws.

In another embodiment, the means to impart a fold includes a spoked wheel having spokes with shaped tips and the method includes feeding the strip around the spoked wheel such that the shaped tips impart a fold to the strip as the wheel rotates.

Preferably, the spoked wheel meshes with another wheel or roller so that the spokes are sandwiched between the shaped tips of the spokes and said other wheel or roller to impart a fold to the strip.

According to another aspect of the invention, there is provided a device for coiling a strip of blisters comprising means for manipulating a strip of blisters so to impart a continuous curvature or a series of discrete creases to the strip as said strip is fed around or through said means.

The means preferably comprises a roller around which a strip is fed. Alternatively, it may comprise two rollers positioned such that the strip follows a curved path as it passes between said rollers.

In one embodiment, the first and second rollers have parallel axes spaced from each other in a direction of movement of the strip, the second roller also having its axis offset from the first axis in a second direction substantially at right angles to the direction of movement of the strip.

The second axis may be offset from the first axis by a distance which is greater than the radius but less than the diameter of the rollers.

In one embodiment, the device comprises a third roller having its axis parallel to but spaced from the axes of the first and second rollers in the direction of movement of the strip such that the second roller is offset from a line extending between the first and second rollers.

The rollers are advantageously configured to simultaneously place opposite sides of the strip in compression and tension, respectively.

In one embodiment, the third roller comprises two coaxial roller portions spaced from each other in an axial direction so as to contact edge portions of the strip.

According to another aspect of the invention, there is provided a device for coiling a strip of blisters comprising means for imparting folds to the strip, each fold extending transversely across the strip and being spaced from its adjacent fold along the length of the strip so that the strip assumes a coiled shape due to said folds.

In one embodiment, the means comprises shaped jaws, said jaws being configured to close on a strip so that a fold is imparted to the strip by the jaws and open to allow the strip to be fed between the jaws.

In another embodiment, the first jaw comprises a mouth and the second jaw comprises a former to press a portion of the strip passing between the jaws into the mouth of said first jaw when the first and second jaws are closed, thereby imparting a fold to the strip.

In an alternative embodiment, the means to impart a fold includes a spoked wheel comprising spokes with shaped tips configured to press against the strip as it is fed around said spoked wheel such that the shaped tips impart a fold to the strip as the wheel rotates.

Preferably, the spoked wheel cooperates with another wheel, roller or surface so that the strip is sandwiched between the shaped tips of the spokes and said other wheel, roller or surface to impart a fold to the strip.

According to another aspect, there is provided a blister strip coiled in accordance with the method of the invention and, preferably, using the device of the invention.

According to another aspect of the invention, there is provided an inhalation device incorporating means for manipulating a used portion of a strip of blisters so to impart a continuous curvature or a series of discrete folds to the strip as said strip is fed around or through said means such that the used portion of the strip assumes a coiled shape within the device.

In a preferred embodiment, said means comprises an indexing wheel for sequentially moving each blister into alignment with a blister piercing member on rotation of the actuator and a blister creasing wheel drivingly coupled to the indexing wheel for rotating the indexing wheel in response to rotation of the actuator, the indexing wheel having spokes shaped to engage the strip passing around the indexing wheel against the blister creasing wheel to impart a fold to the strip.

Preferably, the blister creasing wheel is coupled to the actuator via a clutch mechanism such that the blister creasing wheel rotates in response to rotation of the actuator from the fully open to the closed position to index the blister strip.

In one embodiment, the blister creasing wheel has notches or depressions formed therein such that the spokes of the indexing wheel press the strip into said notches or depressions to create a fold across the strip.

In one embodiment, the blister creasing wheel has a compliant cylindrical surface such that the spokes of the indexing wheel press the strip against said cylindrical surface, deforming said surface along with the strip to create a fold across the strip.

Embodiments of the invention will now be described, by way of example only, and with reference to Figures 3 to 6 of the accompanying drawings, in which:-
FIGURES 1 and 2 are side sectional views of a conventional inhalation device containing a coiled strip of blisters to show how the blisters of a strip are sequentially moved into alignment with a blister piercing station by movement of an actuator from the position shown in Figure 1 to the position shown in Figure 2 which drives an indexing wheel. A piercing head on the actuator pierces the lid of an aligned blister when the actuator is returned to its normal position, as shown in Figure 1a.
FIGURE 3 is a side view of an elongate strip of blisters being fed between rollers of a blister roll-forming rig;
FIGURE 4 is an embodiment to show how a fold can be imparted to the strip between blisters by forming it between a pair of jaws;
FIGURE 5 is an embodiment to show how folds can be formed in the strip by feeding it around a spoked wheel; and
FIGURE 6(a) and 6(b) illustrate an inhalation device incorporating a device for manipulating the used strip to impart folds that cause it to assume a coiled shape. Figure 6(a) shows the device with the actuator closed and, Figure 6(b) shows the device with the actuator in an open position.

Although reference is made above to a specific type of inhalation device, it will be appreciated that the invention is applicable to any inhalation device incorporating a coiled strip of wound blisters each containing doses of an inhalable medicament.

Without any external forces applied thereto by the housing or some other constraint, a coil of blisters that has been wound around a spindle or spool, will loosen, unwind and/or expand radially due to the natural springyness or resilience of the material from which the strip is formed, as described above.

According to the present invention, a strip of blisters is manipulated by a device so that it forms into a coil naturally, rather than being wound up against the resilience or tension in the material that tends to want to keep it open or unwound. If a natural curvature is imparted to the coil, then it will have little or no residual tension and so no tendency to self-expand or unwind. In fact, it has been found that when a coil is formed as a result of imparting a natural curvature to the strip, it will not only retain its coiled shape but will also exhibit resilience that tends to keep it in is closed or coiled state so that if it is partially unwound and then released, it will re-coil or close back up.

To impart a natural tendency for the strip to assume a curved or coiled shape, the strip may be manipulated by feeding it through a path between rollers of a device so as to bend the strip into a continuously curved shape as it is fed therethough. The load applied to the strip by the rollers must be sufficient to cause the strip to curl but must not be so great so as to cause damage to the strip. Of course, it is possible to feed the strip through one set of rollers or multiple sets of rollers or, to feed it through the same set of rollers multiple times so as to impart a sufficient degree of curvature to the strip that results in the formation of a compact coil of the required tightness. It will be appreciated that this forming process is intended to impart a natural curvature to the strip so that its resilience, which tends to keep it flat or straight is removed.

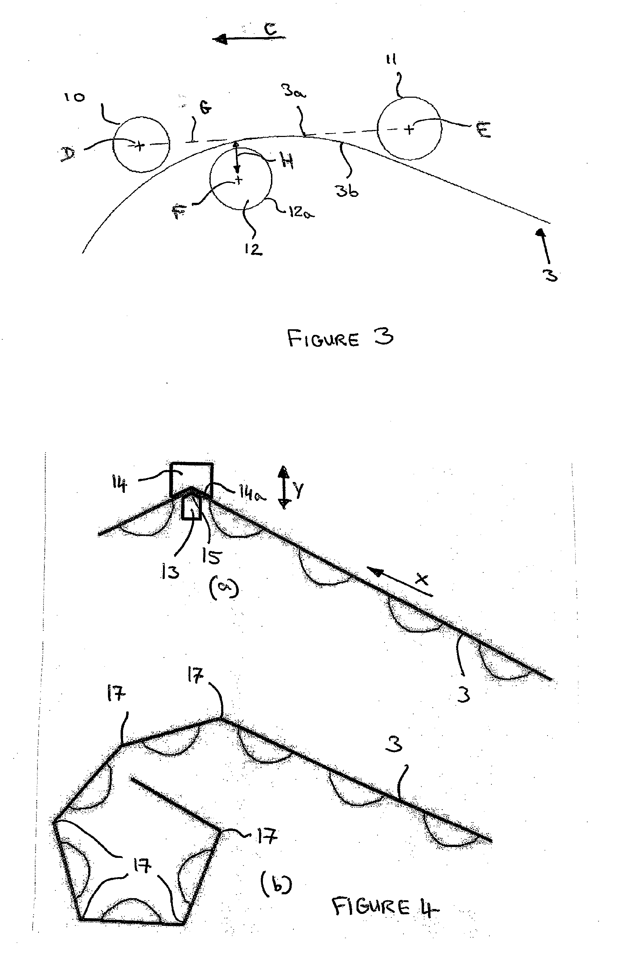

A simplified side view of an elongate strip 3 of blisters (the blister cavities not being shown) being fed between the rollers 10,11,12 of a blister forming rig is shown in Figure 3. Only the rollers 10,11,12 are shown for clarity. The rig comprises three rollers 10,11,12 arranged out-of line or off-set from each other so that a strip 3 is bent into a curved path as it is fed between them. Preferably, at least two of the rollers 10,11,12 are driven to pull the strip 3 through in the general direction indicated by arrow "C" on the drawing. Two spaced apart rollers 10,11 are located so as to engage the outer surface 3a of the strip 3. Each of these rollers 10,11 have their axes "D" and "E" parallel to each other. An intermediate roller 12, also having its axis "F" parallel to the axes of the first two rollers 10,11 is positioned between the first two rollers 10,11 to engage the opposite side of the strip 3b. The axis "F" of the intermediate roller 12 is at least partially offset from a plane "G" extending through the axis D and E of the first two rollers 10,11 in a direction and distance indicated by arrow "H", as shown in Figure 3. It will be appreciated that the exact positions of each of the rollers 10,11,12 can be adjusted to provide the required degree of curvature to the strip 3. Although all the rollers 10,11,12 are of the same diameter, it will be appreciated that rollers of different diameters can also be used. It will also be appreciated that roller 10 could be omitted, so that the strip 3 passes between only two rollers 11, 12, or replaced with some other surface against which the strip slides as it passes through the device.

The first and second rollers 10,11 preferably extend transversely across the entire width of the front 3a surface of the strip 3, whereas the intermediate roller 12 has two roller portions which are spaced apart but coaxial to each other. Only the end surface of one roller portion 12a is shown in Figure 3. This ensures that only the edges of the rear surface 3b of the strip 3 are contacted by the intermediate roller 12, leaving the blister cavities (not shown) untouched as they pass between the two spaced roller portions.

As the strip 3 is fed between the rollers 10,11,12 the front surface 3a of the strip 3 is placed in tension by the first and second rollers 10,11 whereas the opposite surface 3b is placed in compression by the intermediate roller 12. It is this loading that results in the strip 3 assuming a curled shape. As the strip 3 continues to pass through the path between the rollers 10,11,12 it forms into a coil itself due to the curvature imparted to the strip 3 by the rollers 10,11,12.

It is envisaged that the process will be a continuous one, i.e. the entire strip 3 will pass between the rollers 10,11,12. However, it is possible for only part or sections of the strip 3 to be fed between the rollers 10,11,12. For example, one or more of the rollers 10,11,12 could be moved apart whilst the strip 3 continues to be fed between them, thereby only treating a portion of the length of the strip 3. It is also envisaged that one or more of the rollers 10,11,12 can be moved away from the other rollers 10,11,12 to enable a fresh strip 3 to be easily inserted between them. For example, the intermediate roller 12 could be movable into a position in which a straight strip 3 can pass between the rollers 10,11,12. Once in position, the intermediate roller 12 can be moved back into its original position, as shown in Figure 3, prior to feeding the strip 3 through the roller path.

In another embodiment for imparting natural curvature to the strip 3, in which the resilience of the strip 3 which gives it its tendency to spring open or expand is removed, involves subjecting the strip 3 to a bending step in which a series of individual creases, folds or crimps are imparted transversely across the strip 3 between each or several blisters so that the strip 3 assumes a coiled shape following subsequent folds. One way in which this can be achieved is shown in Figure 4. Figure 4a shows how the strip 3 can be passed between a pair of jaws 13,14 , one of which is anvil shaped, whereas the other is a former defining a mouth 14a into which a pointed tip of the anvil shaped jaw is received to impart a fold to the strip pressed between them. Movement of the former 14 and/or the anvil 13 is indicated by arrow Y in Figure 4a. The strip 3 can be indexed forward (in the direction indicated by arrow "X" in Figure 4a) so that folds 17 can be formed in the same way between every blister or between a plurality of groups of blisters to form a coiled strip 3, as shown in Figure 4b.

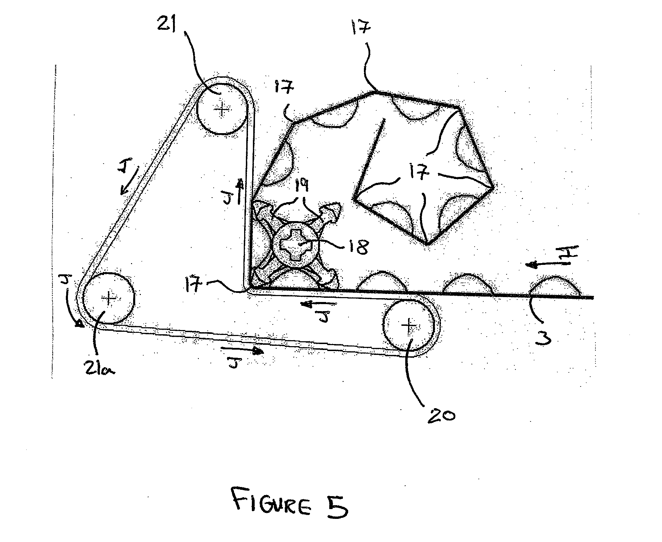

In an alternative embodiment, folds 17 can also be formed in the strip 3 by feeding it (in the direction marked "I" in Figure 5) around a spoked wheel 18 so that the spokes 19 of the wheel 18 form a fold 17 in the strip 3. The spokes 19 may have a pointed or otherwise shaped tip 19a so as to press against and create a crease or fold across the strip 3. The spokes 19 press the strip 3 against another surface, such as a conveyor belt 36 which conveys the strip 3 around the spoked wheel 18. As the strip 3 is sandwiched between the spokes 19 and said surface 36, a fold is thereby imparted to the strip 3. It will be appreciated that this embodiment is rotary and so may more readily lend itself to automation of the process, as opposed to the linear version described with reference to Figure 4.

As shown in Figure 5, a generally flat, elongate strip of blisters 3 is conveyed around a spoked wheel 18. The conveyor 36 extends around guide wheels 20,21 and a conveyor drive wheel 21 a. The position of the guide wheels 20,21 is selected to control the extent to which the strip 3 is wrapped around the spoked wheel 18 so that, as the conveyor 36 is driven in the direction indicated by arrows "J", the spoked wheel 18 rotates and the tip 19a of each spoke 19 imparts a bend or fold extending transversely across the strip 3 between each blister so that the strip assumes a coiled shape on exit from the spoked wheel 18, as shown in Figure 5 due to the folds 17.

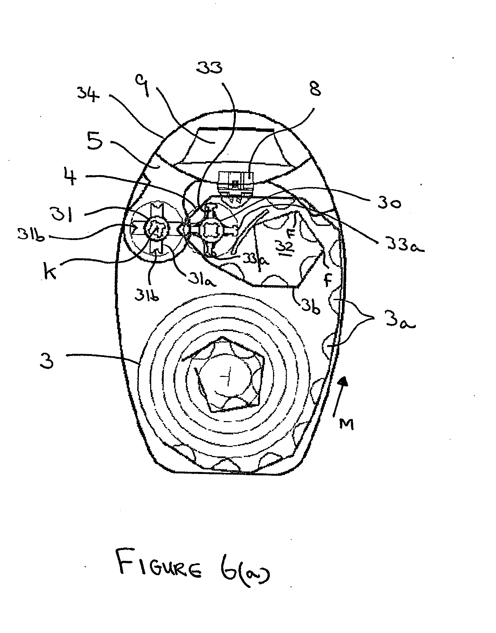

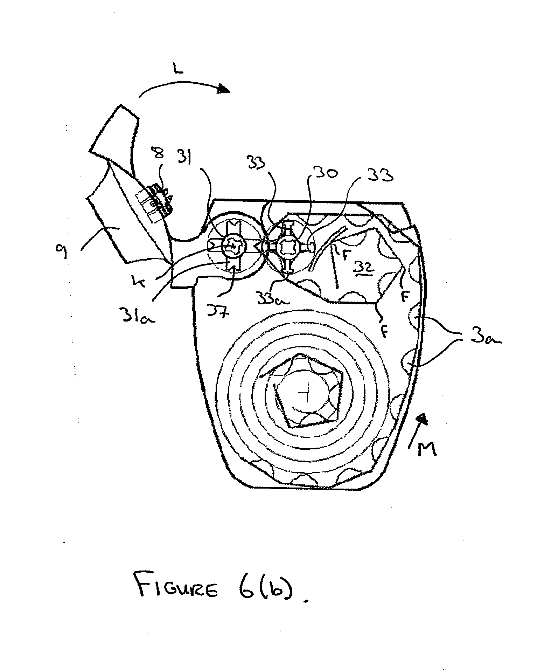

Although the foregoing description generally refers to a winding rig for coiling a strip of blisters prior to insertion into an inhalation device, it is envisaged that one of the described coil forming devices can be directly incorporated into an inhalation device so as to impart a continuous curvature or a series of discrete folds to a used portion of a blister strip that has been uncoiled as it passes over a blister piercing station and around an indexing wheel. An embodiment of inhalation device incorporating means for manipulating a used portion of a blister strip so as to cause it to naturally coil up within the device will now be described with reference to Figures 6(a) and 6(b).

The embodiment of Figure 6 is generally similar to the conventional inhaler of Figure 1 in that it includes a mouthpiece 9, an actuator 5 and an indexing mechanism 4 for sequentially moving each blister 3a into alignment with a blister piercing member 8 in response to actuation of the actuator 5 or, a cap 34 rotatably coupled to the actuator 5 and covering the mouthpiece 9. In Figure 6(a), the actuator 5 is shown in its closed position with a cap 34 over the mouthpiece 9 and, in Figure 6(b), the actuator 5 is shown in is open position after the cap 34 has been opened.

In the embodiment of Figure 6, the indexing mechanism 4 includes an indexing wheel 30 configured to impart individual folds or creases to the strip 3 that extend transversely across the strip 3 between blisters 3a so that the spent portion of the strip 3b naturally forms into a coil as it passes out of the indexing wheel 30.

It will be appreciated that if the indexing wheel 30 is used to form folds or creases across the strip 3, the strip 3 must not be straightened out after emerging from the indexing wheel 30 before being allowed to coil, as it does in the conventional inhalation device shown in Figure 1. Therefore, in the device according to an embodiment of the present invention shown in Figure 6, the layout is arranged such that the spent coil 3b is stored within the housing of the device above the unused coil 3 and close to the folding mechanism, i.e. the spent coil 3b is stored adjacent to the indexing wheel 30 and directly beneath the blister piercing member 8 and between the blister piercing member 8 and the unused coil 3. It is then only the unused coil 3 that is straightened out as it travels up the side of the device from the unused blister coil 3 to the blister piercing member 8.

In addition to the indexing wheel 30, the device according to this embodiment has a blister creasing wheel 31 having spokes 31a whose tips 31b engage with the tips 33a of the spokes 33 of the indexing wheel 30 as the indexing and creasing wheels 30,31 rotate In the version shown, the blister creasing wheel 31 lies coaxial with the axis "K" about which the actuator 5 rotates. The blister creasing wheel 31 is coupled to the actuator 5 via a clutch or ratchet mechanism (not shown) so that the blister creasing wheel 30 rotates (clockwise) only when the actuator 5 is being moved back towards its closed position in the direction of arrow "L" (see Figure 6b). The blister creasing wheel 31 is coupled to the indexing wheel 30 so that the indexing wheel 30 rotates in response to rotation of the blister creasing wheel 31 to index the blister strip. Therefore, indexing of the blister strip 3 occurs on the return stroke of the actuator 5 i.e. from its open to its closed positions, rather than as the actuator is moved from a closed to an open position. The clutch or other mechanism prevents rotation of the blister creasing wheel 31 during rotation of the actuator from its closed to its open position which would otherwise cause the indexing wheen 30 to rotate in a clockwise direction and push the strip backwards towards the unused blister strip chamber.

The clutch or other mechanism causes the blister creasing wheel 31 and actuator 5 to engage when the actuator 5 has reached its fully open limit. This has the advantage that the user can open the actuator 5 almost completely, for cleaning of the mouthpiece 9, and then close it again without indexing the strip 3.

As illustrated in Figure 6, the strip 3 is fed from the coil of unused blisters 3 (shown incomplete for clarity) up the sidewall of the device (in the direction of arrow "M") and past the blister piercing member 8 before being fed around the indexing wheel 30 and into a used coil storage area 32. The tips 33a of the spokes 33 of the indexing wheel 30 and the tips 31b of the spokes 31a of the blister creasing wheel 31 are so shaped that, as the strip 3 passes between the indexing and blister creasing wheels 30, 31, the tip 33a of a spoke 33 of the indexing wheel 30 engages with the tip 31b of a spoke 31a of the blister creasing wheel 31 so that a fold or crease is imparted to the strip, as shown most clearly, by letter "F" in Figure 6, at the point of contact between the tips 31b, 33a. Repeated folds between blisters, caused due to rotation of the blister indexing and creasing wheels 30,31, cause the strip to naturally assume a coiled up shape, as shown by the used portion 3b of the strip that has been fed into the used coil storage area 32.

In one embodiment, the tips 31b of the spokes 31a of the blister creasing wheel 31 may be provided with depressions or notches 37 (see Figure 6b) formed in its surface into which the blister strip is pressed by the tips 33a of the spokes 33 of the indexing wheel to impart a fold to the strip, the tips 33a of the spokes 33 of the indexing wheel 30 may taper towards their ends for this purpose. Alternatively or additionally, the spokes 31a or tips 31b of the blister creasing wheel may be formed from a compliant rubber material

It will be appreciated that the embodiment of Figure 6 is a modification of the embodiment of Figure 4, essentially in that Figure 6 uses a similar jaw arrangement but provides four jaws arranged at 90 degree intervals and so provides a rotary version of the linear version illustrated in Figure 4.

Many modifications and variations of the invention falling within the terms of the following claims will be apparent to those skilled in the art and the foregoing description should be regarded as a description of the preferred embodiments of the invention only. For example, in another unillustrated embodiment, a continuous curvature may be imparted to the strip by feeding the strip through a curved channel so that it assumes a coiled shape on exit from the channel. It will also be appreciated, that the inhalation device incorporating the coil forming device of the invention need not retain used blisters. On the contrary, the used blisters may pass through an opening in the housing of the device for detachment from those blisters that remain in the housing by the patient. In this instance, the blisters may self-coil as they exit the housing. It will also be appreciated that the blister strip may also be provided with lines of weakness between blisters to enable them to be separated or torn more easily. A blister strip of this type is known from the Applicant's earlier application W02006/108876.

A variety of medicaments may be administered alone by using inhalers of the invention. Specific active agents or drugs that may be used include, but are not limited to, agents of one or more of the following classes listed below.
1) Adrenergic agonists such as, for example, amphetamine, apraclonidine, bitolterol, clonidine, colterol, dobutamine, dopamine, ephedrine, epinephrine, ethylnorepinephrine, fenoterol, formoterol, guanabenz, guanfacine, hydroxyamphetamine, isoetharine, isoproterenol, isotharine, mephenterine, metaraminol, methamphetamine, methoxamine, methpentermine, methyldopa, methylphenidate, metaproterenol, metaraminol, mitodrine, naphazoline, norepinephrine, oxymetazoline, pemoline, phenylephrine, phenylethylamine, phenylpropanolamine, pirbuterol, prenalterol, procaterol, propylhexedrine, pseudoephedrine, ritodrine, salbutamol, salmeterol, terbutaline, tetrahydrozoline, tramazoline, tyramine and xylometazoline.
2) Adrenergic antagonists such as, for example, acebutolol, alfuzosin, atenolol, betaxolol, bisoprolol, bopindolol, bucindolol, bunazosin, butyrophenones, carteolol, carvedilol, celiprolol, chlorpromazine, doxazosin, ergot alkaloids, esmolol, haloperidol, indoramin, ketanserin, labetalol, levobunolol, medroxalol, metipranolol, metoprolol, nebivolol, nadolol, naftopidil, oxprenolol, penbutolol, phenothiazines, phenoxybenzamine, phentolamine, pindolol, prazosin, propafenone, propranolol, sotalol, tamsulosin, terazosin, timolol, tolazoline, trimazosin, urapidil and yohimbine.
3) Adrenergic neurone blockers such as, for example, bethanidine, debrisoquine, guabenxan, guanadrel, guanazodine, guanethidine, guanoclor and guanoxan.
4) Drugs for treatment of addiction, such as, for example, buprenorphine.
5) Drugs for treatment of alcoholism, such as, for example, disulfiram, naloxone and naltrexone.
6) Drugs for Alzheimer's disease management, including acetylcholinesterase inhibitors such as, for example, donepezil, galantamine, rivastigmine and tacrin.
7) Anaesthetics such as, for example amethocaine, benzocaine, bupivacaine, hydrocortisone, ketamine, lignocaine, methylprednisolone, prilocaine, proxymetacaine, ropivacaine and tyrothricin.
8) Angiotensin converting enzyme inhibitors such as, for example, captopril, cilazapril, enalapril, fosinopril, imidapril hydrochloride, lisinopril, moexipril hydrochloride, perindopril, quinapril, ramipril and trandolapril.
9) Angiotensin II receptor blockers, such as, for example, candesartan, cilexetil, eprosartan, irbesartan, losartan, medoxomil, olmesartan, telmisartan and valsartan.
10) Antiarrhythmics such as, for example, adenosine, amidodarone, disopyramide, flecainide acetate, lidocaine hydrochloride, mexiletine, procainamide, propafenone and quinidine.
11) Antibiotic and antibacterial agents (including the beta-lactams, fluoroquinolones, ketolides, macrolides, sulphonamides and tetracyclines) such as, for example, aclarubicin, amoxicillin, amphotericin, azithromycin, aztreonam chlorhexidine, clarithromycin, clindamycin, colistimethate, dactinomycin, dirithromycin, doripenem, erythromycin, fusafungine, gentamycin, metronidazole, mupirocin, natamycin, neomycin, nystatin, oleandomycin, pentamidine, pimaricin, probenecid, roxithromycin, sulphadiazine and triclosan.
12) Anti-clotting agents such as, for example, abciximab, acenocoumarol, alteplase, aspirin, bemiparin, bivalirudin, certoparin, clopidogrel, dalteparin, danaparoid, dipyridamole, enoxaparin, epoprostenol, eptifibatide, fondaparin, heparin (including low molecular weight heparin), heparin calcium, lepirudin, phenindione, reteplase, streptokinase, tenecteplase, tinzaparin, tirofiban and warfarin.
13) Anticonvulsants such as, for example, GABA analogs including tiagabine and vigabatrin; barbiturates including pentobarbital; benzodiazepines including alprazolam, chlordiazepoxide, clobazam, clonazepam, diazepam, flurazepam, lorazepam, midazolam, oxazepam and zolazepam; hydantoins including phenytoin; phenyltriazines including lamotrigine; and miscellaneous anticonvulsants including acetazolamide, carbamazepine, ethosuximide, fosphenytoin, gabapentin, levetiracetam, oxcarbazepine, piracetam, pregabalin, primidone, sodium valproate, topiramate, valproic acid and zonisamide.
14) Antidepressants such as, for example, tricyclic and tetracyclic antidepressants including amineptine, amitriptyline (tricyclic and tetracyclic amitryptiline), amoxapine, butriptyline, cianopramine, clomipramine, demexiptiline, desipramine, dibenzepin, dimetacrine, dosulepin, dothiepin, doxepin, imipramine, iprindole, levoprotiline, lofepramine, maprotiline, melitracen, metapramine, mianserin, mirtazapine, nortryptiline, opipramol, propizepine, protriptyline, quinupramine, setiptiline, tianeptine and trimipramine; selective serotonin and noradrenaline reuptake inhibitors (SNRIs) including clovoxamine, duloxetine, milnacipran and venlafaxine; selective serotonin reuptake inhibitors (SSRIs) including citalopram, escitalopram, femoxetine, fluoxetine, fluvoxamine, ifoxetine, milnacipran, nomifensine, oxaprotiline, paroxetine, sertraline, sibutramine, venlafaxine, viqualine and zimeldine; selective noradrenaline reuptake inhibitors (NARIs) including demexiptiline, desipramine, oxaprotiline and reboxetine, noradrenaline and selective serotonin reuptake inhibitors (NASSAs) including mirtazapine; monoamine oxidase inhibitors (MAOIs) including amiflamine, brofaromine, clorgyline, α-ethyltryptamine, etoperidone, iproclozide, iproniazid, isocarboxazid, mebanazine, medifoxamine, moclobemide, nialamide, pargyline, phenelzine, pheniprazine, pirlindole, procarbazine, rasagiline, safrazine, selegiline, toloxatone and tranylcypromine; muscarinic antagonists including benactyzine and dibenzepin; azaspirones including buspirone, gepirone, ipsapirone, tandospirone and tiaspirone; and other antidepressants including acetaphenazine, ademetionine, S-adenosylmethionine, adrafinil, amesergide, amineptine, amperozide, benactyzine, benmoxine, binedaline, bupropion, carbamazepine, caroxazone, cericlamine, cotinine, fezolamine, flupentixol, idazoxan, kitanserin, levoprotiline, lithium salts, maprotiline, medifoxamine, methylphenidate, metralindole, minaprine, nefazodone, nisoxetine, nomifensine, oxaflozane, oxitriptan, phenyhydrazine, rolipram, roxindole, sibutramine, teniloxazine, tianeptine, tofenacin, trazadone, tryptophan, viloxazine and zalospirone.
15) Anticholinergic agents such as, for example, atropine, benzatzopine, biperiden, cyclopentolate, glycopyrrolate, hyoscine, ipratropium bromide, orphenadine hydrochloride, oxitroprium bromide, oxybutinin, pirenzepine, procyclidine, propantheline, propiverine, telenzepine, tiotropium, trihexyphenidyl, tropicamide and trospium.
16) Antidiabetic agents such as, for example, pioglitazone, rosiglitazone and troglitazone.
17) Antidotes such as, for example, deferoxamine, edrophonium chloride, fiumazenil, nalmefene, naloxone, and naltrexone.
18) Anti-emetics such as, for example, alizapride, azasetron, benzquinamide, bestahistine, bromopride, buclizine, chlorpromazine, cinnarizine, clebopride, cyclizine, dimenhydrinate, diphenhydramine, diphenidol, domperidone, dolasetron, dronabinol, droperidol, granisetron, hyoscine, lorazepam, metoclopramide, metopimazine, nabilone, ondansetron, palonosetron, perphenazine, prochlorperazine, promethazine, scopolamine, triethylperazine, trifluoperazine, triflupromazine, trimethobenzamide and tropisetron.
19) Antihistamines such as, for example, acrivastine, astemizole, azatadine, azelastine, brompheniramine, carbinoxamine, cetirizine, chlorpheniramine, cinnarizine, clemastine, cyclizine, cyproheptadine, desloratadine, dexmedetomidine, diphenhydramine, doxylamine, fexofenadine, hydroxyzine, ketotifen, levocabastine, loratadine, mizolastine, promethazine, pyrilamine, terfenadine and trimeprazine.
20) Anti-infective agents such as, for example, antivirals (including nucleoside and non-nucleoside reverse transcriptase inhibitors and protease inhibitors) including aciclovir, adefovir, amantadine, cidofovir, efavirenz, famiciclovir, foscarnet, ganciclovir, idoxuridine, indinavir, inosine pranobex, lamivudine, nelfinavir, nevirapine, oseltamivir, palivizumab, penciclovir, pleconaril, ribavirin, rimantadine, ritonavir, ruprintrivir, saquinavir, stavudine, valaciclovir, zalcitabine, zanamivir, zidovudine and interferons; AIDS adjunct agents including dapsone; aminoglycosides including tobramycin; antifungals including amphotericin, caspofungin, clotrimazole, econazole nitrate, fluconazole, itraconazole, ketoconazole, miconazole, nystatin, terbinafine and voriconazole; anti-malarial agents including quinine; antituberculosis agents including capreomycin, ciprofloxacin, ethambutol, meropenem, piperacillin, rifampicin and vancomycin; beta-lactams including cefazolin, cefmetazole, cefoperazone, cefoxitin, cephacetrile, cephalexin, cephaloglycin and cephaloridine; cephalosporins, including cephalosporin C and cephalothin; cephamycins such as cephamycin A, cephamycin B, cephamycin C, cephapirin and cephradine; leprostatics such as clofazimine; penicillins including amoxicillin, ampicillin, amylpenicillin, azidocillin, benzylpenicillin, carbenicillin, carfecillin, carindacillin, clometocillin, cloxacillin, cyclacillin, dicloxacillin, diphenicillin, heptylpenicillin, hetacillin, metampicillin, methicillin, nafcillin, 2-pentenylpenicillin, penicillin N, penicillin O, penicillin S and penicillin V; quinolones including ciprofloxacin, clinafloxacin, difloxacin, grepafloxacin, norfloxacin, ofloxacine and temafloxacin; tetracyclines including doxycycline and oxytetracycline; miscellaneous anti-infectives including linezolide, trimethoprim and sulfamethoxazole.
21) Anti-neoplastic agents such as, for example, droloxifene, tamoxifen and toremifene.
22) Antiparkisonian drugs such as, for example, amantadine, andropinirole, apomorphine, baclofen, benserazide, biperiden, benztropine, bromocriptine, budipine, cabergoline, carbidopa, eliprodil, entacapone, eptastigmine, ergoline, galanthamine, lazabemide, levodopa, lisuride, mazindol, memantine, mofegiline, orphenadrine, trihexyphenidyl, pergolide, piribedil, pramipexole, procyclidine, propentofylline, rasagiline, remacemide, ropinerole, selegiline, spheramine, terguride and tolcapone.
23) Antipsychotics such as, for example, acetophenazine, alizapride, amisulpride, amoxapine, amperozide, aripiprazole, benperidol, benzquinamide, bromperidol, buramate, butaclamol, butaperazine, carphenazine, carpipramine, chlorpromazine, chlorprothixene, clocapramine, clomacran, clopenthixol, clospirazine, clothiapine, clozapine, cyamemazine, droperidol, flupenthixol, fluphenazine, fluspirilene, haloperidol, loxapine, melperone, mesoridazine, metofenazate, molindrone, olanzapine, penfluridol, pericyazine, perphenazine, pimozide, pipamerone, piperacetazine, pipotiazine, prochlorperazine, promazine, quetiapine, remoxipride, risperidone, sertindole, spiperone, sulpiride, thioridazine, thiothixene, trifluperidol, triflupromazine, trifluoperazine, ziprasidone, zotepine and zuclopenthixol; phenothiazines including aliphatic compounds, piperidines and piperazines; thioxanthenes, butyrophenones and substituted benzamides.
24) Antirheumatic agents such as, for example, diclofenac, heparinoid, hydroxychloroquine and methotrexate, leflunomide and teriflunomide.
25) Anxiolytics such as, for example, adinazolam, alpidem, alprazolam, alseroxlon, amphenidone, azacyclonol, bromazepam, bromisovalum, buspirone, captodiamine, capuride, carbcloral, carbromal, chloral betaine, chlordiazepoxide, clobenzepam, enciprazine, flesinoxan, flurazepam, hydroxyzine, ipsapiraone, lesopitron, loprazolam, lorazepam, loxapine, mecloqualone, medetomidine, methaqualone, methprylon, metomidate, midazolam, oxazepam, propanolol, tandospirone, trazadone, zolpidem and zopiclone.
26) Appetite stimulants such as, for example, dronabinol.
27) Appetite suppressants such as, for example, fenfluramine, phentermine and sibutramine; and anti-obesity treatments such as, for example, pancreatic lipase inhibitors, serotonin and norepinephrine re-uptake inhibitors, and anti-anorectic agents.
28) Benzodiazepines such as, for example, alprazolam, bromazepam, brotizolam, chlordiazepoxide, clobazam, clonazepam, clorazepate, demoxepam, diazepam, estazolam, flunitrazepam, flurazepam, halazepam, ketazolam, loprazolam, lorazepam, lormetazepam, medazepam, midazolam, nitrazepam, nordazepam, oxazepam, prazepam, quazepam, temazepam and triazolam.
29) Bisphosphonates such as, for example, alendronate sodium, sodium clodronate, etidronate disodium, ibandronic acid, pamidronate disodium, isedronate sodium, tiludronic acid and zoledronic acid.
30) Blood modifiers such as, for example, cilostazol and dipyridamol, and blood factors.
31) Cardiovascular agents such as, for example, acebutalol, adenosine, amiloride, amiodarone, atenolol, benazepril, bisoprolol, bumetanide, candesartan, captopril, clonidine, diltiazem, disopyramide, dofetilide, doxazosin, enalapril, esmolol, ethacrynic acid, flecanide, furosemide, gemfibrozil, ibutilide, irbesartan, labetolol, losartan, lovastatin, metolazone, metoprolol, mexiletine, nadolol, nifedipine, pindolol, prazosin, procainamide, propafenone, propranolol, quinapril, quinidine, ramipril, sotalol, spironolactone, telmisartan, tocainide, torsemide, triamterene, valsartan and verapamil.
32) Calcium channel blockers such as, for example, amlodipine, bepridil, diltiazem, felodipine, flunarizine, gallopamil, isradipine, lacidipine, lercanidipine, nicardipine, nifedipine, nimodipine and verapamil.
33) Central nervous system stimulants such as, for example, amphetamine, brucine, caffeine, dexfenfluramine, dextroamphetamine, ephedrine, fenfluramine, mazindol, methyphenidate, modafmil, pemoline, phentermine and sibutramine.
34) Cholesterol-lowering drugs such as, for example, acipimox, atorvastatin, ciprofibrate, colestipol, colestyramine, bezafibrate, ezetimibe, fenofibrate, fluvastatin, gemfibrozil, ispaghula, nictotinic acid, omega-3 triglycerides, pravastatin, rosuvastatin and simvastatin.
35) Drugs for cystic fibrosis management such as, for example, Pseudomonas aeruginosa infection vaccines (eg Aerugen™), alpha 1-antitripsin, amikacin, cefadroxil, denufosol, duramycin, glutathione, mannitol, and tobramycin.
36) Diagnostic agents such as, for example, adenosine and aminohippuric acid.
37) Dietary supplements such as, for example, melatonin and vitamins including vitamin E.
38) Diuretics such as, for example, amiloride, bendroflumethiazide, bumetanide, chlortalidone, cyclopenthiazide, furosemide, indapamide, metolazone, spironolactone and torasemide.
39) Dopamine agonists such as, for example, amantadine, apomorphine, bromocriptine, cabergoline, lisuride, pergolide, pramipexole and ropinerole.
40) Drugs for treating erectile dysfunction, such as, for example, apomorphine, apomorphine diacetate, moxisylyte, phentolamine, phosphodiesterase type 5 inhibitors, such as sildenafil, tadalafil, vardenafil and yohimbine.
41) Gastrointestinal agents such as, for example, atropine, hyoscyamine, famotidine, lansoprazole, loperamide, omeprazole and rebeprazole.
42) Hormones and analogues such as, for example, cortisone, epinephrine, estradiol, insulin, Ostabolin-C, parathyroid hormone and testosterone.
43) Hormonal drugs such as, for example, desmopressin, lanreotide, leuprolide, octreotide, pegvisomant, protirelin, salcotonin, somatropin, tetracosactide, thyroxine and vasopressin.
44) Hypoglycaemics such as, for example, sulphonylureas including glibenclamide, gliclazide, glimepiride, glipizide and gliquidone; biguanides including metformin; thiazolidinediones including pioglitazone, rosiglitazone, nateglinide, repaglinide and acarbose.
45) Immunoglobulins.
46) Immunomodulators such as, for example, interferon (e.g. interferon beta-1a and interferon beta-1b) and glatiramer.
47) Immunosupressives such as, for example, azathioprine, cyclosporin, mycophenolic acid, rapamycin, sirolimus and tacrolimus.
48) Mast cell stabilizers such as, for example, cromoglycate, iodoxamide, nedocromil, ketotifen, tryptase inhibitors and pemirolast.
49) Drugs for treatment of migraine headaches such as, for example, almotriptan, alperopride, amitriptyline, amoxapine, atenolol, clonidine, codeine, coproxamol, cyproheptadine, dextropropoxypene, dihydroergotamine, diltiazem, doxepin, ergotamine, eletriptan, fluoxetine, frovatriptan, isometheptene, lidocaine, lisinopril, lisuride, loxapine, methysergide, metoclopramide, metoprolol, nadolol, naratriptan, nortriptyline, oxycodone, paroxetine, pizotifen, pizotyline, prochlorperazine propanolol, propoxyphene, protriptyline, rizatriptan, sertraline, sumatriptan, timolol, tolfenamic acid, tramadol, verapamil, zolmitriptan, and non-steroidal anti-inflammatory drugs.
50) Drugs for treatment of motion sickness such as, for example, diphenhydramine, promethazine and scopolamine.
51) Mucolytic agents such as N-acetylcysteine, ambroxol, amiloride, dextrans, heparin, desulphated heparin, low molecular weight heparin and recombinant human DNase.
52) Drugs for multiple sclerosis management such as, for example, bencyclane, methylprednisolone, mitoxantrone and prednisolone.
53) Muscle relaxants such as, for example, baclofen, chlorzoxazone, cyclobenzaprine, methocarbamol, orphenadrine, quinine and tizanidine.
54) NMDA receptor antagonists such as, for example, mementine.
55) Nonsteroidal anti-inflammatory agents such as, for example, aceclofenac, acetaminophen, alminoprofen, amfenac, aminopropylon, amixetrine, aspirin, benoxaprofen, bromfenac, bufexamac, carprofen, celecoxib, choline, cinchophen, cinmetacin, clometacin, clopriac, diclofenac, diclofenac sodium, diflunisal, ethenzamide, etodolac, etoricoxib, fenoprofen, flurbiprofen, ibuprofen, indomethacin, indoprofen, ketoprofen, ketorolac, loxoprofen, mazipredone, meclofenamate, mefenamic acid, meloxicam, nabumetone, naproxen, nimesulide, parecoxib, phenylbutazone, piroxicam, pirprofen, rofecoxib, salicylate, sulindac, tiaprofenic acid, tolfenamate, tolmetin and valdecoxib.
56) Nucleic-acid medicines such as, for example, oligonucleotides, decoy nucleotides, antisense nucleotides and other gene-based medicine molecules.
57) Opiates and opioids such as, for example, alfentanil, allylprodine, alphaprodine, anileridine, benzylmorphine, bezitramide, buprenorphine, butorphanol, carbiphene, cipramadol, clonitazene, codeine, codeine phosphate, dextromoramide, dextropropoxyphene, diamorphine, dihydrocodeine, dihydromorphine, diphenoxylate, dipipanone, fentanyl, hydromorphone, L-alpha acetyl methadol, levorphanol, lofentanil, loperamide, meperidine, meptazinol, methadone, metopon, morphine, nalbuphine, nalorphine, oxycodone, papaveretum, pentazocine, pethidine, phenazocine, pholcodeine, remifentanil, sufentanil, tramadol, and combinations thereof with an antiemetic.
58) Opthalmic preparations such as, for example, betaxolol and ketotifen.
59) Osteoporosis preparations such as, for example, alendronate, estradiol, estropitate, raloxifene and risedronate.
60) Other analgesics such as, for example, apazone, benzpiperylon, benzydamine, caffeine, cannabinoids, clonixin, ethoheptazine, flupirtine, nefopam, orphenadrine, pentazocine, propacetamol and propoxyphene.
61) Other anti-inflammatory agents such as, for example, B-cell inhibitors, p38 MAP kinase inhibitors and TNF inhibitors.
62) Phosphodiesterase inhibitors such as, for example, non-specific phosphodiesterase inhibitors including theophylline, theobromine, IBMX, pentoxifylline and papaverine; phosphodiesterase type 3 inhibitors including bipyridines such as milrinone, amrinone and olprinone; imidazolones such as piroximone and enoximone; imidazolines such as imazodan and 5-methyl-imazodan; imidazo-quinoxalines; and dihydropyridazinones such as indolidan and LY181512 (5-(6-oxo-1,4,5,6-tetrahydro-pyridazin-3-yl)-1,3-dihydro-indol-2-one); dihydroquinolinone compounds such as cilostamide, cilostazol, and vesnarinone; motapizone; phosphodiesterase type 4 inhibitors such as cilomilast, etazolate, rolipram, oglemilast, roflumilast, ONO 6126, tolafentrine and zardaverine, and including quinazolinediones such as nitraquazone and nitraquazone analogs; xanthine derivatives such as denbufylline and arofylline; tetrahydropyrimidones such as atizoram; and oxime carbamates such as filaminast; and phosphodiesterase type 5 inhibitors including sildenafil, zaprinast, vardenafil, tadalafil, dipyridamole, and the compounds described in WO 01/19802, particularly (S)-2-(2-hydroxymethyl-l-pyrrolidinyl)-4-(3-chloro-4-methoxy-benzylamino)-5-[N-(2-pyrimidinylmethyl)carbamoyl]pyrimidine, 2-(5,6,7,8-tetrahydro-1, 7-naphthyridin-7-y1)-4-(3-chloro-4-methoxybenzylamino)-5-[N-(2-morpholinoethyl)carbamoyl]-pyrimidine, and (S)-2-(2-hydroxymethyl-1-pyrrolidinyl)-4-(3-chloro-4-methoxy-benzylamino)-5-[N-(1,3,5-trimethyl-4-pyrazolyl)carbamoyl]-pyrimidine).
63) Potassium channel modulators such as, for example, cromakalim, diazoxide, glibenclamide, levcromakalim, minoxidil, nicorandil and pinacidil.
64) Prostaglandins such as, for example, alprostadil, dinoprostone, epoprostanol and misoprostol.
65) Respiratory agents and agents for the treatment of respiratory diseases including bronchodilators such as, for example, the β₂-agonists bambuterol, bitolterol, broxaterol, carmoterol, clenbuterol, fenoterol, formoterol, indacaterol, levalbuterol, metaproterenol, orciprenaline, picumeterol, pirbuterol, procaterol, reproterol, rimiterol, salbutamol, salmeterol, terbutaline and the like; inducible nitric oxide synthase (iNOS) inhibitors; the antimuscarinics ipratropium, ipratropium bromide, oxitropium, tiotropium, glycopyrrolate and the like; the xanthines aminophylline, theophylline and the like; adenosine receptor antagonists, cytokines such as, for example, interleukins and interferons; cytokine antagonists and chemokine antagonists including cytokine synthesis inhibitors, endothelin receptor antagonists, elastase inhibitors, integrin inhibitors, leukotrine receptor antagonists, prostacyclin analogues, and ablukast, ephedrine, epinephrine, fenleuton, iloprost, iralukast, isoetharine, isoproterenol, montelukast, ontazolast, pranlukast, pseudoephedrine, sibenadet, tepoxalin, verlukast, zafirlukast and zileuton.
66) Sedatives and hypnotics such as, for example, alprazolam, butalbital, chlordiazepoxide, diazepam, estazolam, flunitrazepam, flurazepam, lorazepam, midazolam, temazepam, triazolam, zaleplon, zolpidem, and zopiclone.
67) Serotonin agonists such as, for example, 1-(4-bromo-2,5-dimethoxyphenyl)-2-aminopropane, buspirone, m-chlorophenylpiperazine, cisapride, ergot alkaloids, gepirone, 8-hydroxy-(2-N,N-dipropylamino)-tetraline, ipsaperone, lysergic acid diethylamide, 2-methyl serotonin, mezacopride, sumatriptan, tiaspirone, trazodone and zacopride.
68) Serotonin antagonists such as, for example, amitryptiline, azatadine, chlorpromazine, clozapine, cyproheptadine, dexfenfluramine, R(+)-α-(2,3-dimethoxyphenyl)-1-[2-(4-fluorophenyl)ethyl]-4-piperidine-methanol, dolasetron, fenclonine, fenfluramine, granisetron, ketanserin, methysergide, metoclopramide, mianserin, ondansetron, risperidone, ritanserin, trimethobenzamide and tropisetron.
69) Steroid drugs such as, for example, alcometasone, beclomethasone, beclomethasone dipropionate, betamethasone, budesonide, butixocort, ciclesonide, clobetasol, deflazacort, diflucortolone, desoxymethasone, dexamethasone, fludrocortisone, flunisolide, fluocinolone, fluometholone, fluticasone, fluticasone proprionate, hydrocortisone, methylprednisolone, mometasone, nandrolone decanoate, neomycin sulphate, prednisolone, rimexolone, rofleponide, triamcinolone and triamcinolone acetonide.
70) Sympathomimetic drugs such as, for example, adrenaline, dexamfetamine, dipirefin, dobutamine, dopamine, dopexamine, isoprenaline, noradrenaline, phenylephrine, pseudoephedrine, tramazoline and xylometazoline.
71) Nitrates such as, for example, glyceryl trinitrate, isosorbide dinitrate and isosorbide mononitrate.
72) Skin and mucous membrane agents such as, for example, bergapten, isotretinoin and methoxsalen.
73) Smoking cessation aids such as, for example, bupropion, nicotine and varenicline.
74) Drugs for treatment of Tourette's syndrome such as, for example, pimozide.
75) Drugs for treatment of urinary tract infections such as, for example, darifenicin, oxybutynin, propantheline bromide and tolteridine.
76) Vaccines.
77) Drugs for treating vertigo such as, for example, betahistine and meclizine.
78) Therapeutic proteins and peptides such as acylated insulin, glucagon, glucagon-like peptides, exendins, insulin, insulin analogues, insulin aspart, insulin detemir, insulin glargine, insulin glulisine, insulin lispro, insulin zinc, isophane insulins, neutral, regular and insoluble insulins, and protamine zinc insulin.
79) Anticancer agents such as, for example, anthracyclines, doxorubicin, idarubicin, epirubicin, methotrexate, taxanes, paclitaxel, docetaxel, cisplatin, vinca alkaloids, vincristine and 5-fluorouracil.
80) Pharmaceutically acceptable salts or derivatives of any of the foregoing.

It should be noted that drugs listed above under a particular indication or class may also find utility in other indications. A plurality of active agents can be employed in the practice of the present invention. An inhaler according to the invention may also be used to deliver combinations of two or more different active agents or drugs. Specific combinations of two medicaments which may be mentioned include combinations of steroids and β₂-agonists. Examples of such combinations are beclomethasone and formoterol; beclomethasone and salmeterol; fluticasone and formoterol; fluticasone and salmeterol; budesonide and formoterol; budesonide and salmeterol; flunisolide and formoterol; flunisolide and salmeterol; ciclesonide and formoterol; ciclesonide and salmeterol; mometasone and formoterol; and mometasone and salmeterol. Specifically, inhalers according to the invention may also be used to deliver combinations of three different active agents or drugs.

It will be clear to a person of skill in the art that, where appropriate, the active agents or drugs may be linked to a carrier molecule or molecules and/or used in the form of prodrugs, salts, as esters, or as solvates to optimise the activity and/or stability of the active agent or drug.

Anticholinergic agents are referred to above (see No. 15). It is also envisaged that the pharmaceutical composition may comprise one or more, preferably one, anticholinergic 1, optionally in combination with a pharmaceutically acceptable excipient.

The anticholinergic 1 can be selected from the group consisting of
a) tiotropium salts 1a,
b) compounds of formula 1c 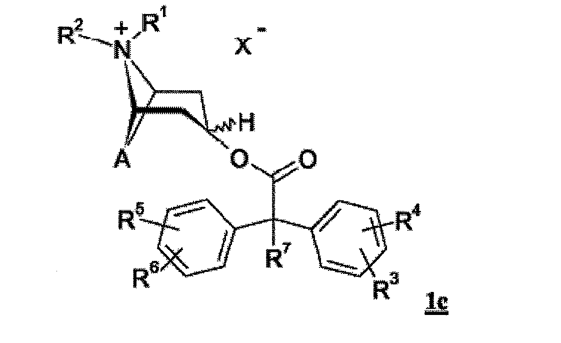 wherein
   A denotes a double-bonded group selected from among 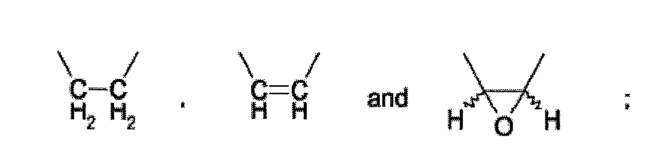
   **X⁻** denotes an anion with a single negative charge, preferably an anion selected from the group consisting of fluoride, chloride, bromide, iodide, sulphate, phosphate, methanesulphonate, nitrate, maleate, acetate, citrate, fumarate, tartrate, oxalate, succinate, benzoate and p-toluenesulphonate,
   R¹ and R² which may be identical or different denote a group selected from among methyl, ethyl, n-propyl and iso-propyl, which may optionally be substituted by hydroxy or fluorine, preferably unsubstituted methyl;
   R³, R⁴, R⁵ and R⁶, which may be identical or different, denote hydrogen, methyl, ethyl, methyloxy, ethyloxy, hydroxy, fluorine, chlorine, bromine, CN, CF₃ or NO₂;
   R⁷ denotes hydrogen, methyl, ethyl, methyloxy, ethyloxy, -CH₂-F, -CH₂-CH₂-F, -O-CH₂-F, -O-CH₂-CH₂-F, -CH₂-OH, -CH₂-CH₂-OH, CF₃, -CH₂-OMe,-CH₂-CH₂-OMe, -CH₂-OEt, -CH₂-CH₂-OEt, -O-COMe, -O-COEt, -Q-COCF₃, -Q-COCF₃, fluorine, chlorine or bromine;
c) compounds of formula 1d 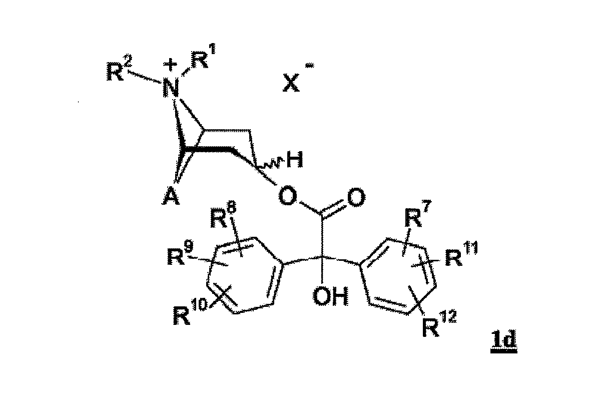 wherein
   A, **X⁻** , R¹ and R² may have the meanings as mentioned hereinbefore and wherein R⁷, R⁸, R⁹, R¹⁰, R¹¹ and R¹² , which may be identical or different, denote hydrogen, methyl, ethyl, methyloxy, ethyloxy, hydroxy, fluorine, chlorine, bromine, CN, CF₃ or NO₂, with the proviso that at least one of the groups R⁷, R⁸, R⁹, R¹⁰, R¹¹ and R¹² is not hydrogen,
d) compounds of formula 1e 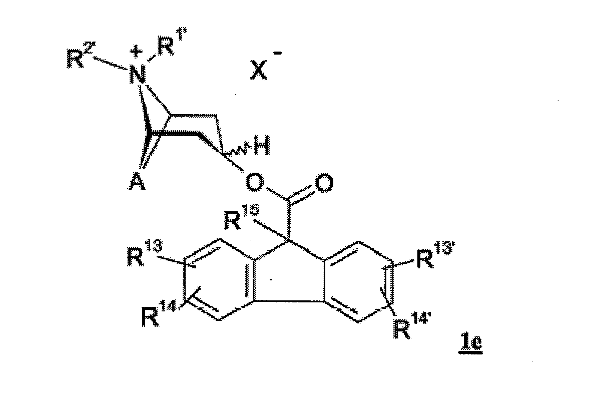 wherein A and **X⁻** may have the meanings as mentioned hereinbefore, and wherein R¹⁵ denotes hydrogen, hydroxy, methyl, ethyl, -CF₃, CHF₂ or fluorine;
   R^{1'} and R^{2'} which may be identical or different denote C₁-C5-alkyl which may optionally be substituted by C₃-C₆-cycloalkyl, hydroxy or halogen, or
   R^{1'} and R^{2'} together denote a -C₃-C₅-alkylene-bridge;
   R¹³, R¹⁴, R^{13'} and R^{14'} which may be identical or different denote hydrogen, -C₁-C₄-aky!, -C₁-C₄-alkyloxy, hydroxy, -CF₃, -CHF₂, CN, NO₂ or halogen,
e) compounds of formula 1f 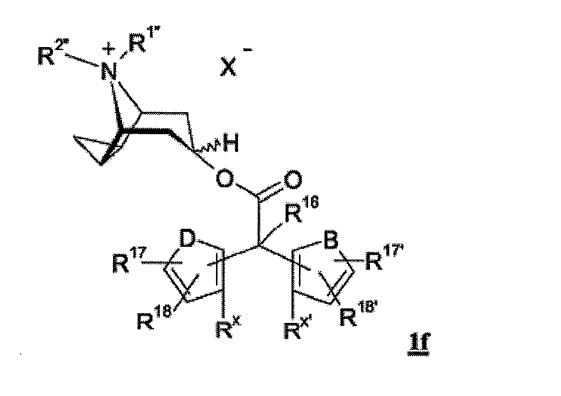 wherein **X⁻** may have the meanings as mentioned hereinbefore, and wherein
   D and B which may be identical or different, preferably identical, denote -O, -S, -NH, - CH₂, -CH=CH, or -N(C₁-C₄-alkyl)-;
   R¹⁶ denotes hydrogen, hydroxy, -C₁-C₄alkyl, -C₁-C₄-alkyloxry, -C₁- C₄ - alkylene-Halogen, -O-C₁-C₄ alkylene-halogen, -C₁ -C₄-alkylene-OH, -CF₃ , CHF₂, -C₁-C₄-alkylene-C₁-C₄alkyloxy, -O- COC₁ -C₄-alkyl, -O-COC₁ -C₄ -alkylene-halogen, -C₁-C₄-akylene-C₃-C₆-cycloakyl, -O-COCF₃ or halogen;
   R^{1"} and R^{2"} which may be identical or different, denote -C₁-C₅-alkyl, which may optionally be substituted by -C₃-C₆-cycloalkyl, hydroxy or halogen, or
   R^{1"} and R^{2"} together denote a -C3-C5-alkylene bridge;
   R¹⁷, R¹⁸, R^{17'} and R^{18'}, which may be identical or different, denote hydrogen, C₁-C₄-alkyl, C₁-C₄-alkyloxy, hydroxy, -CF₃, -CHF₂, CN, NO₂ or halogen;
   R^{x} and R^{x'} which may be identical or different, denote hydrogen, C₁-C₄-alkyl, C₁-C₄-alkyloxy, hydroxy, -CF₃, -CHF₂, CN, NO₂ or halogen or
   R^{x} and R^{x'} together denote a single bond or a bridging group selected from among the bridges -O, -S, -NH, -CH₂, -CH₂-CH₂-, -N(C₁-C₄-alkyl), -CH(C₁-C₄₋alkyl)- and -C(C₁-C₄-alkyl)₂, and
f) compounds of formula 1g 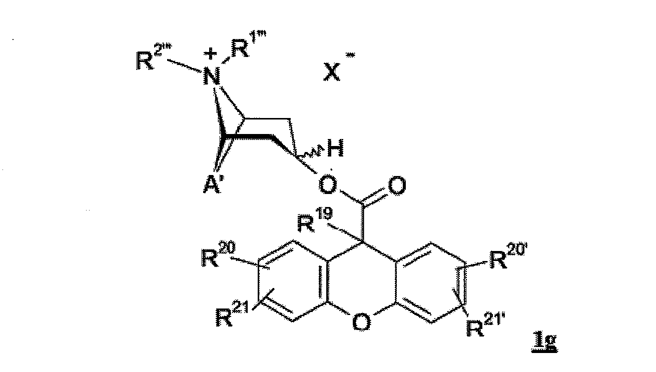 wherein **X⁻** may have the meanings as mentioned hereinbefore, and wherein A' denotes a double-bonded group selected from among 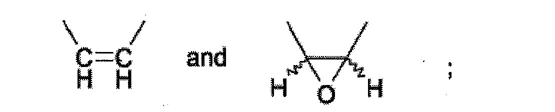 R¹⁹ denotes hydroxy, methyl, hydroxymethyl, ethyl, -CF₃, CHF₂ or fluorine;
   R^{1'''} and R^{2'''} which may be identical or different denote C₁-C₅-alkyl which may optionally be substituted by C₃-C₆-cycloalkyl, hydroxy or halogen, or
   R^{1'''} and R^{2'''} together denote a -C₃-C₅-alkylene-bridge;
   R²⁰, R²¹, R^{20'} and R^{21'} which may be identical or different denote hydrogen, -C₁ -C₄-alkyl, -C₁-C₄-alkyloxy, hydroxy, -CF₃, -CHF₂, CN, NO₂ or halogen.

The compounds of formula 1c are known in the art (WO 02/32899).

In a preferred embodiment of the invention the method comprises administration of compounds of formula 1c, wherein
- **X⁻**: denotes bromide;
R¹ and R² which may be identical or different denote a group selected from methyl and ethyl, preferably methyl;
R³, R⁴, R⁵ and R⁶, which may be identical or different, denote hydrogen, methyl, methyloxy, chlorine or fluorine;
R⁷ denotes hydrogen, methyl or fluorine, optionally together with a pharmaceutically acceptable excipient.

Of particular importance are compounds of general formula 1c, wherein A denotes a double-bonded group selected from among

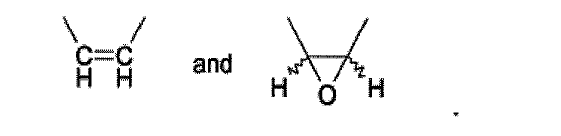

The compounds of formula 1c, may optionally be administered in the form of the individual optical isomers, mixtures of the individual enantiomers or racemates thereof.

Of particular importance within a method according to the invention are the following compounds of formula 1c:
tropenol 2,2-diphenylpropionic acid ester methobromide,
scopine 2,2-diphenylpropionic acid ester methobromide,
scopine 2-fluoro-2,2-diphenylacetic acid ester methobromide and
tropenol 2-fluoro-2,2-diphenylacetic acid ester methobromide.

The compounds of formula 1d are known in the art (WO 02/32898).

In a preferred embodiment of the invention the method comprises administration of compounds of formula 1d, wherein
- A: denotes a double-bonded group selected from among

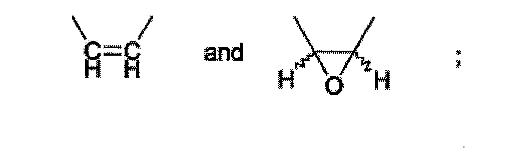

- **X⁻**: denotes bromide;
R¹ and R² which may be identical or different denote methyl or ethyl, preferably methyl;
R⁷, R⁸, R⁹, R¹⁰, R¹¹ and R¹², which may be identical or different, denote hydrogen, fluorine, chlorine or bromine, preferably fluorine with the proviso that at least one of the groups R⁷, R⁸, R⁹, R¹⁰, R¹¹ and R¹² not hydrogen, optionally together with a pharmaceutically acceptable excipient.

Of particular importance within the method according to the invention are the following compounds of formula 1d:
tropenol 3,3',4,4'-tetrafluorobenzilic acid ester methobromide,
scopine 3,3',4,4'-tetrafluorobenzilic acid ester methobromide,
scopine 4,4'-difluorobenzilic acid ester methobromide,
tropenol 4,4'-difluorobenzilic acid ester methobromide,
scopine 3,3'-difluorobenzilic acid ester methobromide, and
tropenol 3,3'-difluorobenzilic acid ester methobromide.

The pharmaceutical compositions according to the invention may contain the compounds of formula 1d optionally in the form of the individual optical isomers, mixtures of the individual enantiomers or racemates thereof.

The compounds of formula 1e are known in the art (WO 03/064419).

In a preferred embodiment of the invention the method comprises administration of compounds of formula 1e, wherein
- A: denotes a double-bonded group selected from among 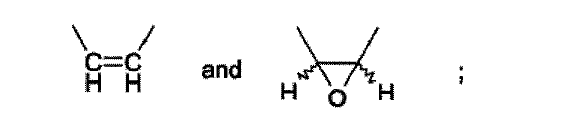
- **X⁻**: denotes an anion selected from among chloride, bromide and methanesulphonate, preferably bromide;
R¹⁵ denotes hydroxy, methyl or fluorine, preferably methyl or hydroxy;
R^{1'} and R^{2'} which may be identical or different represent methyl or ethyl, preferably methyl;
R¹³, R¹⁴, R^{13'} and R^{14'} which may be identical or different represent hydrogen, -CF₃, - CHF₂ or fluorine, preferably hydrogen or fluorine, optionally together with a pharmaceutically acceptable excipient.

In another preferred embodiment of the invention the method comprises administration of compounds of formula 1e, wherein
- A: denotes a double-bonded group selected from among 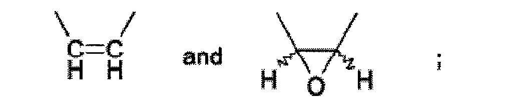
- **X⁻**: denotes bromide;
R¹⁵ denotes hydroxy or methyl, preferably methyl;
R^{1'} and R^{2'} which may be identical or different represent methyl or ethyl, preferably methyl;
R¹³, R¹⁴, R^{13'} and R^{14'} which may be identical or different represent hydrogen or fluorine, optionally together with a pharmaceutically acceptable excipient.

Of particular importance within the method according to the invention are the following compounds of formula 1e:
tropenol 9-hydroxy-fluorene-9-carboxylate methobromide ;
tropenol 9-fluoro-fluorene-9-carboxylate methobromide;
scopine 9-hydroxy-fluorene-9-carboxylate methobromide;
scopine 9-fluoro-fluorene-9-carboxylate methobromide;
tropenol 9-methyl-fluorene-9-carboxylate methobromide;
scopine 9-methyl-fluorene-9-carboxylate methobromide.

The pharmaceutical compositions according to the invention may contain the compounds of formula 1e optionally in the form of the individual optical isomers, mixtures of the individual enantiomers or racemates thereof.

The compounds of formula 1f are known in the art (WO 03/064418).

In another preferred embodiment of the invention the method comprises administration of compounds of formula 1f wherein
**X⁻** denotes chloride, bromide, or methanesulphonate, preferably bromide;
D and B which may be identical or different, preferably identical, denote -O, -S, -NH or -CH=CH-;
R¹⁶denotes hydrogen, hydroxy, -C₁-C₄-alkyl, -C₁-C₄alkyloxy, -CF₃, -CHF₂, fluorine, chlorine or bromine;
R^{1"} and R^{2"} which may be identical or different, denote C₁ -C₄-alky, which may optionally be substituted by hydroxy, fluorine, chlorine or bromine, or
R^{1"} and R^{2"} together denote a -C₃-C₄-alkylene-bridge;
R¹⁷, R¹⁸, R^{17'} and R^{18'}, which may be identical or different, denote hydrogen, C₁-C₄ - alkyl, C₁-C₄-akyloxy, hydroxy, -CF₃, -CHF₂, CN, NO₂, fluorine, chlorine or bromine; R^{x} and R^{x'} which may be identical or different, denote hydrogen, C₁-C₄-akyl, C₁-C₄-alkyloxy, hydroxy, -CF₃, -CHF₂, CN, NO₂, fluorine, chlorine or bromine or
R^{x} and R^{x'} together denote a single bond or a bridging group selected from among the bridges -O, -S, -NH- and -CH₂- , optionally together with a pharmaceutically acceptable excipient.

In another preferred embodiment of the invention the method comprises administration of compounds of formula 1f, wherein
- **X⁻**: denotes chloride, bromide, or methanesulphonate, preferably bromide;
- D and B: which may be identical or different, preferably identical, denote -S or-CH=CH-;
- R¹⁶: denotes hydrogen, hydroxy or methyl;
R^{1"} and R^{2"} which may be identical or different, denote methyl or ethyl;
R¹⁷, R¹⁸, R^{17'} and R^{18'}, which may be identical or different, denote hydrogen, -CF₃ or fluorine, preferably hydrogen;
R^{x} and R^{x'} which may be identical or different, denote hydrogen, -CF₃ or fluorine, preferably hydrogen or
R^{x} and R^{x'} together denote a single bond or the bridging group -O-, optionally together with a pharmaceutically acceptable excipient.

In another preferred embodiment of the invention the method comprises administration of compounds of formula 1f wherein
- **X⁻**: denotes bromide;
- D and B: denote -CH=CH-;
- R¹⁶: denotes hydrogen, hydroxy or methyl;
R^{1"} and R^{2"} denote methyl;
R¹⁷, R¹⁸, R^{17'} and R^{18'} , which may be identical or different, denote hydrogen or fluorine, preferably hydrogen;
R^{x} and R^{x'} which may be identical or different, denote hydrogen or fluorine, preferably hydrogen or
R^{x} and R^{x'} together denote a single bond or the bridging group -O-, optionally together with a pharmaceutically acceptable excipient.

Of particular importance within the method according to the invention are the following compounds of formula 1f:
cyclopropyltropine benzilate methobromide;
cyclopropyltropine 2,2-diphenylpropionate methobromide;
cyclopropyltropine 9-hydroxy-xanthene-9-carboxylate methobromide;
cyclopropyltropine 9-methyl-fluorene-9-carboxylate methobromide; cyclopropyltropine 9-methyl-xanthene-9-carboxylate methobromide; cyclopropyltropine 9-hydroxy-fluorene-9-carboxylate methobromide; cyclopropyltropine methyl 4,4'-difluorobenzilate methobromide.

The pharmaceutical compositions according to the invention may contain the compounds of formula 1f optionally in the form of the individual optical isomers, mixtures of the individual enantiomers or racemates thereof.

The compounds of formula 1g are known in the art (WO 03/064417).

In another preferred embodiment of the invention the method comprises administration of compounds of formula 1g wherein
- A': denotes a double-bonded group selected from among

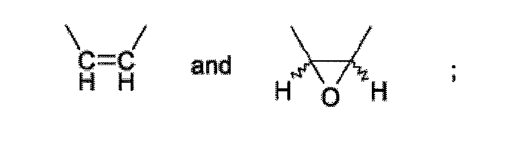

- **X⁻**: denotes chloride, bromide or methanesulphonate, preferably bromide;
R¹⁹ denotes hydroxy or methyl;
R^{1'''} and R^{2'''} which may be identical or different represent methyl or ethyl, preferably methyl;
R²⁰, R²¹, R^{20'} and R^{21'} which may be identical or different represent hydrogen, -CF₃, - CHF₂ or fluorine, preferably hydrogen or fluorine, optionally together with a pharmaceutically acceptable excipient.

In another preferred embodiment of the invention the method comprises administration of compounds of formula 1g wherein
- A': denotes a double-bonded group selected from among

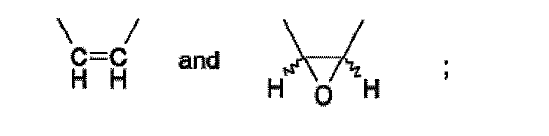

- **X⁻**: denotes bromide;
R¹⁹ denotes hydroxy or methyl, preferably methyl;
R^{1'''} and R^{2'''} which may be identical or different represent methyl or ethyl, preferably methyl;
R³, R⁴, R^{3'} and R^{4'} which may be identical or different represent hydrogen or fluorine, optionally together with a pharmaceutically acceptable excipient.

Of particular importance within the method according to the invention are the following compounds of formula 1g:
tropenol 9-hydroxy-xanthene-9-carboxylate methobromide;
scopine 9-hydroxy-xanthene-9-carboxylate methobromide;
tropenol 9-methyl-xanthene-9-carboxylate methobromide;
scopine 9-methyl-xanthene-9-carboxylate methobromide;
tropenol 9-ethyl-xanthene-9-carboxylate methobromide;
tropenol 9-difluoromethyl-xanthene-9-carboxylate methobromide;
scopine 9-hydroxymethyl-xanthene-9-carboxylate methobromide.

The pharmaceutical compositions according to the invention may contain the compounds of formula 1g optionally in the form of the individual optical isomers, mixtures of the individual enantiomers or racemates thereof.

The alkyl groups used, unless otherwise stated, are branched and unbranched alkyl groups having 1 to 5 carbon atoms. Examples include: methyl, ethyl, propyl or butyl. The groups methyl, ethyl, propyl or butyl may optionally also be referred to by the abbreviations Me, Et, Prop or Bu. Unless otherwise stated, the definitions propyl and butyl also include all possible isomeric forms of the groups in question. Thus, for example, propyl includes n- propyl and iso-propyl, butyl includes iso-butyl, sec. butyl and tert. -butyl, etc.

The cycloalkyl groups used, unless otherwise stated, are alicyclic groups with 3 to 6 carbon atoms. These are the cyclopropyl, cyclobutyl, cyclopentyl and cyclohexyl groups.
According to the invention cyclopropyl is of particular importance within the scope of the present invention.

The alkylene groups used, unless otherwise stated, are branched and unbranched double- bonded alkyl bridges with 1 to 5 carbon atoms. Examples include: methylene, ethylene, propylene or butylene.

The alkylene-halogen groups used, unless otherwise stated, are branched and unbranched double-bonded alkyl bridges with 1 to 4 carbon atoms which may be mono-, di- or trisubstituted, preferably disubstituted, by a halogen. Accordingly, unless otherwise stated, the term alkylene-OH groups denotes branched and unbranched double-bonded alkyl bridges with 1 to 4 carbon atoms which may be mono-, di- or trisubstituted, preferably monosubstituted, by a hydroxy.

The alkyloxy groups used, unless otherwise stated, are branched and unbranched alkyl groups with 1 to 5 carbon atoms which are linked via an oxygen atom. The following may be mentioned, for example: methyloxy, ethyloxy, propyloxy or butyloxy. The groups methyloxy, ethyloxy, propyloxy or butyloxy may optionally also be referred to by the abbreviations MeO, EtO, PropO or BuO. Unless otherwise stated, the definitions propyloxy and butyloxy also include all possible isomeric forms of the groups in question. Thus, for example, propyloxy includes n-propyloxy and iso-propyloxy, butyloxy includes iso-butyloxy, sec. butyloxy and tert. -butyloxy, etc. The word alkoxy may also possibly be used within the scope of the present invention instead of the word alkyloxy. The groups methyloxy, ethyloxy, propyloxy or butyloxy may optionally also be referred to as methoxy, ethoxy, propoxy or butoxy.

The alkylene-alkyloxy groups used, unless otherwise stated, are branched and unbranched double-bonded alkyl bridges with 1 to 5 carbon atoms which may be mono-, di- or trisubstituted, preferably monosubstituted, by an alkyloxy group.

The -O-CO-alkyl groups used, unless otherwise stated, are branched and unbranched alkyl groups with 1 to 4 carbon atoms which are bonded via an ester group. The alkyl groups are bonded directly to the carbonylcarbon of the ester group. The term -O-CO-alkyl-halogen group should be understood analogously. The group -O-CO-CF₃ denotes trifluoroacetate.

Within the scope of the present invention halogen denotes fluorine, chlorine, bromine or iodine. Unless otherwise stated, fluorine and bromine are the preferred halogens. The group CO denotes a carbonyl group.

The inhalation device according to the invention comprises the compounds of formula 1 preferably in admixture with a pharmaceutically acceptable excipient to form a powder mixture. The following pharmaceutically acceptable excipients may be used to prepare these inhalable powder mixtures according to the invention: monosaccharides (e.g. glucose or arabinose), disaccharides (e.g. lactose, saccharose, maltose, trehalose), oligo- and polysaccharides (e.g. dextrane), polyalcohols (e.g. sorbitol, mannitol, xylitol), salts (e.g. sodium chloride, calcium carbonate) or mixtures of these excipients with one another. Preferably, mono- or disaccharides are used, while the use of lactose or glucose is preferred, particularly, but not exclusively, in the form of their hydrates. For the purposes of the invention, lactose and trehalose are the particularly preferred excipients, while lactose, preferably in form of its monohydrate is most particularly preferred.

The compounds of formula 1 may be used in the form of their racemates, enantiomers or mixtures thereof. The separation of enantiomers from the racemates may be carried out using methods known in the art (e.g. by chromatography on chiral phases, etc.).

Optionally, the inhalation device according to the invention contains plural of doses of a medicament in powder form that contains, beside one compound of formula 1, another active ingredient.

Preferably the additional active ingredient is a beta₂ agonists 2 which is selected from the group consisting of albuterol, bambuterol, bitolterol, broxaterol, carbuterol, clenbuterol, fenoterol, formoterol, hexoprenaline, ibuterol, isoetharine, isoprenaline, levosalbutamol, mabuterol, meluadrine, metaproterenol, orciprenaline, pirbuterol, procaterol, reproterol, rimiterol, ritodrine, salmeterol, salmefamol, soterenot, sulphonterol, tiaramide, terbutaline, tolubuterol, CHF-1035, HOKU-81, KUL-1248, 3-(4- {6-[2-Hydroxy-2-(4-hydroxy-3- hydi-oxymethyl-phenyl)-ethylamino]-hexyloxy}-butyl)-benzenesulfoneamide, 5-[2-(5,6- Diethyl-indan-2-ylamino)-1-hydroxy-ethyl]-8-hydroxy-1H-quinolin-2-one , 4-hydroxy-7- [2- { [2- { [3 -(2-phenylethoxy)propyl] sulphonyl} ethyl] -amino} ethyl] -2(3H)-benzothiazolone , 1 -(2-fluoro-4-hydroxyphenyl)-2-[4-(1-benzimidazolyl)-2-methyl-2-butylamino]ethanol, 1 - [3 -(4-methoxybenzyl-amino)-4-hydroxyphenyl] -2- [4-( 1 -benzimidazolyl)-2-methyl-2-butylamino]ethanol , 1 -[2H-5-hydroxy-3-oxo-4H-1,4-benzoxazin-8-yl]-2-[3-(4-N,N-dimethylaminophenyl)-2-methyl-2-propylamino]ethanol 1-[2H-5-hydroxy-3-oxo-4H-1,4- benzoxazin-8-yl]-2-[3-(4-methoxyphenyl)-2-methyl-2-propylamino]ethanol , 1 -[2H-5- hydroxy-3 -0X0-4H- 1 ,4-benzoxazin-8-yl] -2- [3 -(4-n-butyloxyphenyl)-2-methyl-2-propylamino]ethanol , 1 - [2H-5-hydroxy-3 -oxo-4H- 1 ,4-benzoxazin-8-yl] -2- {4- [3 - (4- methoxyphenyl)-1,2,4-tziazol-3-yl]-2-methyl-2-butylamino} ethanol , 5-hydroxy-8-(1-hydroxy-2-isopropylaminobutyl)-2H-1,4-benzoxazin-3-(4H)-one,1-(4-amino-3-chloro-5-trifluormethylphenyl)-2-tert.-butylamino)ethanol and 1 -(4-ethoxycarbonylamino-3-cyano- 5-fluorophenyl)-2-(tert.-butylamino)ethanol, optionally in the form of the racemates, the enantiomers, the diastereomers and optionally the pharmacologically acceptable acid addition salts and the hydrates thereof.

According to the instant invention more preferred beta₂ agonists 2 are selected from the group consisting of bambuterol, bitolterol, carbuterol, clenbuterol, fenoterol, formoterol, hexoprenaline, ibuterol, pirbuterol, procaterol, reproterol, salmeterol, sulphonterol, terbutaline, tolubuterol, 3-(4- {6-[2-Hydroxy-2-(4-hydroxy-3-hydroxymethyl-phenyl)- ethylamino]-hexyloxy} -butyl)-benzenesulfoneamide, 5-[2-(5,6-Diediyl-indan-2-ylamino)-1-hydroxy-ethyl]-8-hydroxy-1H-quinolin-2-one, 4-hydroxy-7-[2-{[2-{[3-(2- phenylethoxy)propyl] sulphonyl} ethyl]-amino} ethyl]-2(3H)-benzothiazolone 1-(2-fluoro- 4-hydroxyphenyl)-2-[4-(1-benzimidazolyl)-2-methyl-2-butylamino]ethanol , 1 -[3-(4- methoxybenzyl-amino)-4-hydroxyphenyl] -2- [4-(1-benzimidazolyl)-2-methyl-2- butylamino] ethanol, 1-[2H-5-hydroxy-3-oxo-4H-1,4-benzoxazin-8-yl]-2-[3-(4-N,N- dimethylaminophenyl)-2-methyl-2-propylamino]ethanol , 1-[2H-5-hydroxy-3-oxo-4H-1,4- benzoxazin-8-yl]-2-[3-(4-methoxyphenyl)-2-methyl-2-propylamino]ethanol, -[2H-5- hydroxy-3 -0X0-4H- 1 ,4-benzoxazin-8-yl] -2- [3 -(4-n-butyloxyphenyl)-2-methyl-2- propylamino]ethanol 1 - [2H-5-hydroxy-3 -oxo-4H- 1 ,4-benzoxazin-8-yl] -2- {4- [3 -(4- methoxyphenyl)-1,2,4-triazol-3-yl]-2-methyl-2-butylamino}ethanol, 5-hydroxy-8-(1- hydroxy-2-isopropylaminobutyl)-2H-1,4-benzoxazin-3-(4H)-one,1-(4-amino-3-chloro-5- trifluormethylphenyl)-2-tert.-butylamino)ethanol and 1 -(4-ethoxycarbonylamino-3-cyano- 5-fluorophenyl)-2-(tert.-butylamino)ethanol, optionally in the form of the racemates, the enantiomers, the diastereomers and optionally the pharmacologically acceptable acid addition salts and the hydrates thereof.

More preferably, the betamimetics 2 used as within the compositions according to the invention are selected from among fenoterol, formoterol, salmeterol, 3-(4-{6-[2-Hydroxy- 2-(4-hydroxy-3-hydroxymethyl-phenyl)-ethylamino]-hexyloxy}-butyl)-benzenesulfoneamide, 5-[2-(5,6-Diethyl-indan-2-ylamino)-1-hydroxy-ethyl]-8-hydroxy-1H-quinolin-2-one, 1 -[3-(4-methoxybenzyl-amino)-4-hydroxyphenyl]-2-[4-(1-benzimidazolyl)-2-methyl-2-butylamino] ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1 - [2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl] -2- {4- [3 -(4-methoxyphenyl)- 1 ,2,4-triazol-3 -yl] -2-methyl-2-butylamino} ethanol , optionally in the form of the racemates, the enantiomers, the diastereomers and optionally the pharmacologically acceptable acid addition salts thereof, and the hydrates thereof. Of the betamimetics mentioned above the compounds formoterol, salmeterol, 3-(4-{6-[2-Hydroxy-2-(4-hydroxy-3-hydroxymethylphenyl)-ethylamino]- hexyloxy}-butyl)-benzenesulfoneamide, and 5-[2-(5,6-Diethyl-indan-2-ylamino)-1- hydroxy-ethyl]-8-hydroxy-1H-quinolin-2-one are particularly preferred, optionally in the form of the racemates, the enantiomers, the diastereomers and optionally the pharmacologically acceptable acid addition salts thereof, and the hydrates thereof. Of the betamimetics mentioned above the compounds formoterol and salmeterol are particularly preferred, optionally in the form of the racemates, the enantiomers, the diastereomers and optionally the pharmacologically acceptable acid addition salts thereof, and the hydrates thereof.

Examples of pharmacologically acceptable acid addition salts of the betamimetics 2 according to the invention are the pharmaceutically acceptable salts which are selected from among the salts of hydrochloric acid, hydrobromic acid, sulphuric acid, phosphoric acid, methanesulphonic acid, acetic acid, fumaric acid, succinic acid, lactic acid, citric acid, tartaric acid, 1-hydroxy-2-naphthalenecarboxylic acid, 4-phenylcinnamic acid, 5-(2.4- difluorophenyl)salicylic acid or maleic acid. If desired, mixtures of the abovementioned acids may also be used to prepare the salts 2.

According to the invention, the salts of the betamimetics 2 selected from among the hydrochloride, hydrobromide, sulphate, phosphate, fumarate, methanesulphonate, 4-phenylcinnamate, 5-(2.4-difluorophenyl)salicylate, maleate and xinafoate are preferred. Particularly preferred are the salts of 2 in the case of salmeterol selected from among the hydrochloride, sulphate, 4-phenylcinnamate, 5-(2.4-difluorophenyl)salicylate and xinafoate, of which the 4-phenylcinnamate, 5-(2.4-difluorophenyl)salicylate and especially xinafoate are particularly important. Particularly preferred are the salts of 2 in the case of formoterol selected from the hydrochloride, sulphate and fumarate, of which the hydrochloride and fumarate are particularly preferred, such as formoterol fumarate.

Salts of salmeterol, formoterol, 3-(4-{6-[2-Hydroxy-2-(4-hydroxy-3-hydroxymethylphenyl)-ethylamino]-hexyloxy} -butyl)-benzenesulfoneamide, and 5-[2-(5,6-Diethyl-indan- 2-ylamino)-1-hydroxy-ethyl]-8-hydroxy-1H-quinolin-2-one , are preferably used as the betamimetics 2 according to the invention. Of particular importance are salmeterol and formoterol salts. Any reference to the term betamimetics 2 also includes a reference to the relevant enantiomers or mixtures thereof. In the pharmaceutical compositions according to the invention, the compounds 2 may be present in the form of their racemates, enantiomers or mixtures thereof. The separation of the enantiomers from the racemates may be carried out using methods known in the art (e.g. by chromatography on chiral phases, etc.) If the compounds 2 are used in the form of their enantiomers, it is particularly preferable to use the enantiomers in the R configuration at the C-OH group.

Optionally, the inhalation device according to the invention contains plural of doses of a medicament in powder form that contains beside one compound of formula 1 a steroid 3 as another active ingredient.

In such medicament combinations the steroid 3 is preferably selected from among prednisolone, prednisone , butixocortpropionate, RPR- 106541, flunisolide , beclomethasone , triamcinolone , budesonide , fluticasone , mometasone , ciclesonide , rofleponide , ST- 126 , dexamethasone , (S)-fluoromethyl 6α,9α-difluoro-17α-[(2-furanylcarbonyl)oxy] - 11 [beta]-hydroxy- 16α-methyl-3 -oxo-androsta- 1 ,4-diene- 17β-carbothionate , (S)-(2-oxo-tetrahydro-furan-3S-yl)6α,9α-difluoro-1 1 β-hydroxy- 16α-methyl-3 -oxo- 17α-propionyloxy-androsta- 1 ,4-diene- 17β-carbothionate, and etiprednol- dichloroacetate (BNP- 166), optionally in the form of the racemates, enantiomers or diastereomers thereof and optionally in the form of the salts and derivatives thereof, the solvates and/or hydrates thereof.

In particularly preferred medicament combinations the steroid 3 is selected from the group comprising flunisolide , beclomethasone , triamcinolone , budesonide , fluticasone , mometasone , ciclesonide , rofleponide , ST- 126 , dexamethasone , (S)-fluoromethyl 6α,9α-difluoro- 1 Iα- [(2-furanylcarbonyl)oxy] - 11 β-hydroxy-16α-methyl-3 -oxo-androsta-1,4-diene-17β-carbothionate, (S)-(2-oxo-tetrahydro-furan-3S-yl)6α,9α-difluoro-11β- hydroxy- 16α-methyl-3 -oxo- 17α-propionyloxy-androsta- 1 ,4-diene- 17β-carbothionate , and etiprednol-dichloroacetate , optionally in the form of the racemates, enantiomers or diastereomers thereof and optionally in the form of the salts and derivatives thereof, the solvates and/or hydrates thereof.

In particularly preferred medicament combinations the steroid 3 is selected from the group comprising budesonide , fluticasone , mometasone , ciclesonide , (S)-fluoromethyl 6α,9α-difluoro- 1 Iα- [(2-furanylcarbonyl)oxy] - 11 β-hydroxy- 16α-methyl-3 -oxo-androsta- 1 ,A- diene-17β-carbothionate , and etiprednol-dichloroacetate , optionally in the form of the racemates, enantiomers or diastereomers thereof and optionally in the form of the salts and derivatives thereof, the solvates and/or hydrates thereof.

Any reference to steroids 3 includes a reference to any salts or derivatives, hydrates or solvates thereof which may exist. Examples of possible salts and derivatives of the steroids 3 may be: alkali metal salts, such as for example sodium or potassium salts, sulphobenzoates, phosphates, isonicotinates, acetates, propionates, dihydrogen phosphates, palmitates, pivalates or furcates.

Optionally, the inhalation device according to the invention contains plural of doses of a medicament on powder form that contains beside one compound of formula 1 additionally both, one of the betamimetics 2 mentioned hereinbefore and one of the steroids 3 mentioned hereinbefore.

According to one aspect, there is provided an inhalation device according to the invention, wherein each blister contains a pharmaceutical composition in powder form wherein the pharmaceutical composition comprises one or more, preferably one, compound of formula 1.

Within the scope of the inhalable powders according to the invention the excipients have a maximum average particle size of up to 250µm, preferably between 10 and 150µm, most preferably between 15 and 80µm. It may sometimes seem appropriate to add finer excipient fractions with an average particle size of 1 to 9µm to the excipients mentioned above. These finer excipients are also selected from the group of possible excipients listed hereinbefore. Finally, in order to prepare the inhalable powders according to the invention, micronised active substance I-, and optionally 2 and/or 3, preferably with an average particle size of 0.5 to 10µm, more preferably from 1 to 6µm, is added to the excipient mixture. Processes for producing the inhalable powders according to the invention by grinding and micronising and finally mixing the ingredients together are known from the prior art.

For the methods of preparing the pharmaceutical compositions in powder form reference may be made to the disclosure of WO 02/30390, WO 03/017970, or WO 03/017979 for example. The disclosure of WO 02/30390, WO 03/017970, and WO 03/017979 is herby incorporated by reference into the instant patent application in its entirety.

As an example, the pharmaceutical compositions according to the invention may be obtained by the method described below.

First, the excipient and the active substance are placed in a suitable mixing container. The active substance used has an average particle size of 0.5 to 10 µm, preferably 1 to 6 µm, most preferably 2 to 5 µm. The excipient and the active substance are preferably added using a sieve or a granulating sieve with a mesh size of 0.1 to 2 mm, preferably 0.3 to 1 mm, most preferably 0.3 to 0.6 mm. Preferably, the excipient is put in first and then the active substance is added to the mixing container. During this mixing process the two components are preferably added in batches. It is particularly preferred to sieve in the two components in alternate layers. The mixing of the excipient with the active substance may take place while the two components are still being added. Preferably, however, mixing is only done once the two components have been sieved in layer by layer.

If after being chemically prepared the active substance used in the process described above is not already obtainable in a crystalline form with the particle sizes mentioned earlier, it can be ground up into the particle sizes which conform to the abovementioned parameters (so-called micronising).

## Claims

1. A method of coiling a strip of blisters, the method including the step of feeding a preformed strip of blisters around or through means to manipulate the strip so as to impart a continuous curvature or a series of discrete creases to the strip that allow the strip to assume a coiled shape.

2. A method of coiling a strip of blisters according to claim 1, including the step of feeding said strip around a roller.

3. A method of coiling a strip of blisters according to claim 2, wherein the step of feeding the strip around a roller includes the step of feeding it between curved surfaces of two rollers positioned such that opposite sides of the strip are placed in compression and tension, respectively.

4. A method according to claim 3, including the step of feeding the strip under two rollers having their axes parallel but spaced from each other and, over a third roller positioned between the two spaced rollers.

5. A method according to claim 4, including the step of positioning the third roller so that its axis is offset from a line extending between the axis of the first and second rollers so that a strip passing under the first roller must follow a curved path over the second roller.

6. A method according to claim 4 or claim 5, including the step of configuring the third roller so as to contact only the edges of the strip.

7. A method according to claim 1, wherein the step of feeding a preformed strip of blisters around or through means to manipulate the strip comprises feeding said strip through means to impart a series of discrete creases extending transversely across the strip and allowing the strip to naturally assume a coiled shape due to said series of discrete creases.

8. A method according to claim 7, including the step of using said means to impart individual creases between each blister of the strip.

9. A method according to claim 8, including the step of using said means to impart individual creases between several blisters of the strip.

10. A method according to claim 8, including the step of using said means to impart creases between groups of blisters.

11. A method according to any of claims 7 to 10, wherein means comprises shaped jaws and the method includes controlling the jaws to close on a strip so that a crease is imparted to the strip by the jaws, the method further including opening the jaws to allow the strip to be fed between the jaws.

12. A method according to any of claims 7 to 10, wherein the means to impart a crease includes a spoked wheel having spokes with shaped tips and the method includes feeding the strip around the spoked wheel such that the shaped tips impart a crease to the strip as the wheel rotates.

13. A method according to claim 12, wherein the spoked wheel meshes with another wheel or roller so that the strip is sandwiched between the shaped tips of the spokes and said other wheel or roller to impart a crease to the strip.

14. A method according to any preceding claim, wherein the step of feeding the strip around or through means to manipulate the strip comprises feeding it through means to impart plastic deformation to the strip.

15. A device for coiling a strip of blisters comprising means for manipulating a strip of blisters so to impart a continuous curvature or a series of discrete creases to the strip as said strip is fed around or through said means such that the strip naturally assumes a coiled shape.

16. A device according to claim 14, wherein the means comprises a roller around which a strip is fed.

17. A device according to claim 16, comprising two rollers positioned such that the strip follows a curved path as it passes between said rollers.

18. A device according to claim 17, wherein the first and second rollers have parallel axes spaced from each other in a direction of movement of the strip, the second roller also having its axis offset from the first axis in a second direction substantially at right angles to the direction of movement of the strip.

19. A device according to claim 18, wherein the second axis is offset from the first axis by a distance which is greater than the radius but less than the diameter of the rollers.

20. A device according to any of claims 17 to 19, comprising a third roller having its axis parallel to but spaced from the axes of the first and second rollers in the direction of movement of the strip such that the second roller is offset from a line extending between the first and second rollers.

21. A device according to any of claims 17 to 20, wherein said rollers are configured to simultaneously place opposite sides of the strip in compression and tension, respectively.

22. A device according to claim 21, wherein the third roller comprises two coaxial roller portions spaced from each other in an axial direction so as to contact edge portions of the strip.

23. A device for coiling a strip of blisters comprising means for imparting a series of discrete creases to the strip, each crease extending transversely across the strip and being spaced from its adjacent crease along the length of the strip so that the strip assumes a coiled shape due to said series of discrete creases

24. A device according to claim 23, wherein means comprises shaped jaws, said jaws being configured to close on a strip so that a discrete crease is imparted to the strip by the jaws and open to allow the strip to be fed between the jaws.

25. A device according to claim 24, wherein a first jaw comprises a mouth and the second jaw comprises a former to press a portion of the strip passing between the jaws into the mouth of said first jaw when the first and second jaws are closed, thereby imparting a crease to the strip..

26. A device according to claim 23, wherein the means to impart a crease includes a spoked wheel comprising spokes with shaped tips configured to press against the strip as it is fed around said spoked wheel such that the shaped tips impart a crease to the strip as the wheel rotates.

27. A device according to claim 26, wherein the spoked wheel cooperates with another wheel, roller or surface so that the strip is sandwiched between the shaped tips of the spokes and said other wheel, roller or surface to impart a crease to the strip.

28. A device according to any of claims 15 to 27, wherein the means for manipulating the strip comprises means for plastically deforming the strip.

29. A blister strip coiled in accordance with the method of any of claims 1 to 14.

30. A blister strip according to claim 29, coiled using the device of any of claims 15 to 28.

31. An inhalation device incorporating a blister strip according to claim 29.

32. An inhalation device according to claim 31, the blister strip having been coiled using a device of any of claims 15 to 28.

33. An inhalation device incorporating means for manipulating a used portion of a strip of blisters so to impart a continuous curvature or a series of discrete creases to the strip as said strip is fed through said means such that the used portion of the strip assumes a coiled shape

34. An inhalation device according to claim 33, wherein said means comprises an indexing wheel for sequentially moving each blister into alignment with a blister piercing member on rotation of the actuator and a blister creasing wheel, the indexing and blister piercing wheels engaging to impart a series of discrete folds to a strip passing therebetween.

35. An inhalation device according to claim 34, wherein said indexing and blister creasing wheels have spokes with shaped tips, the tips of the spokes engaging during rotation to impart a crease to the strip held therebetween.

36. An inhalation device according to claim 35, wherein the tip of each spoke of the blister creasing wheel has notches or depressions formed therein, the spokes of the indexing wheel being configured to press the strip into said notches or depressions to form a crease in the strip.

37. An inhalation device according to any of claims 34 to 36, wherein the blister creasing wheel is drivingly coupled to the indexing wheel for rotating the indexing wheel in response to rotation of the actuator.

38. An inhalation device according to claim 37, wherein the blister creasing wheel is coupled to the actuator via a clutch mechanism such that the blister creasing wheel rotates in response to rotation of the actuator in one direction.

39. An inhalation device according to claim 38, wherein the blister creasing wheel rotates in response to rotation of the actuator from the open to the closed position to index the blister strip.
